# EUROPEAN PATENT APPLICATION

(11) **EP 1 839 699 A1**
(43) Date of publication of application: **03.10.2007**
(21) Application number: 05819651.0
(22) Date of filing: 22.12.2005
(51) Int. Cl.: A61M 39/02

(54) **CONNECTOR, TUBE ASSEMBLY, INFUSION TUBE SET, AND CONTAINER FOR MEDICAL USE**

(30) Priority: 22.12.2004 JP 2004372238; 13.01.2005 JP 2005006913
(71) Applicant: TERUMO KABUSHIKI KAISHA, Tokyo 151-0072 (JP)
(72) Inventor: HISHIKAWA, Yoshinori, Terumo Kabushiki Kaisha, Nakakoma-gun, Yamanashi 4093853 (JP); YOKOTA, Takayuki, Terumo Kabushiki Kaisha, Nakakoma-gun, Yamanashi 4093853 (JP)
(74) Representative: TBK-Patent
(86) International application number: PCT/JP2005/023574
(87) International publication number: WO 2006/068211

(57) **Abstract**

A connector is constructed from a male connector having a cavity and a female connector having a cavity to which the other male connector similar to the male connector can be connected. The connector has a male lock section and a female lock section to which the other male lock section similar to the male lock section can be coupled. When the connector is in a locked state where the female lock section and the other male lock section are coupled, the female connector and the other male connector are connected such that the cavities are communicated with each other to enable liquid to flow.

## Description

### TECHNICAL FIELD

The present invention relates to a connector, a tube assembly, an infusion tube set, and a container for medical use.

### BACKGROUND ART

In medical systems that require the connection of passages for liquids used for infusion, blood transfusion, nutrition dosing, or the like, connection and disconnection of the liquid passages (circuits) must be conducted as necessary, when a liquid medicine, blood, liquid food, or the like, is caused to flow in a sustained or temporary fashion. In such a situation, a connector for connecting the liquid passages to each other is mounted in the course of the fluid circuit.

A typical example of such a connector is a three-way cock (connector). The connector includes one male-type connection section and two female-type connection sections. When forming a circuit using a plurality of such connectors, the circuit connection (formation) is carried out by fitting a male-type connection section on one side and a female-type connection section on another side. In this case, fixation is conducting using a so-called lure lock, in order to maintain the fitting between the male-type connection section and the female-type connection section. A lure lock implies that a threaded engagement is formed between a female screw and a male screw.

However, fixation by means of a lure lock has problems in that a plurality of connectors must be rotated due to the threaded engagement (rotation) formed between the female screw and the male screw. Therefore, it is difficult to fix the directions of the connectors. As a result, tubes connected to the connectors may become twisted.

In addition, there is also the problem that, due to rotation of the connectors, threaded engagement between the female screw and the male screw might become released, i.e., the lure lock might become released unintentionally.

For coping with such problems, a connector assembly may be provided with a multiple cock, having a plurality of connectors connected preliminarily (i.e., detachably mounted) on a single base (base plate).

In such a connector assembly, however, the base plate cannot be used when another connector is added to the plurality of preliminarily arranged connectors. Therefore, the aforementioned problems of twisting of tubes and unintentional release of the lure lock occur again.

Meanwhile, as a countermeasure to this problem, a connector assembly in which a single connector (cock) is fixed on a single base plate (plate) is known. (For example, refer to Japanese Laid-Open Patent Publication No. 7-67968).

In this connector assembly, a fitting between the male-type connection section on one side and the female-type connection section on the other side is maintained by means of a connection between the plates. Therefore, when relative positional accuracy (dimensional accuracy) between the connector and the plate is low, for example, positional deviations can occur between adjacent connectors, such that the connectors cannot be connected accurately. In addition, if the adjacent connectors are not connected securely, fittings between the male-type connection section on one side and the female-type connection section on the other side are easily released, or in other words, the male-type connection section and the female-type connection section cannot be connected in a manner that enables a liquid to flow therethrough. In addition, poor operability occurs when connecting and disconnecting the two connector assemblies to and from each other.

### DISCLOSURE OF THE INVENTION

An object of the present invention is to provide a connector, a tube assembly, an infusion tube set, and a container for medical use, wherein the connector can be placed in a locked state easily and assuredly for enabling a liquid to flow.

In order to attain the above objects, the present invention includes:
a connector including a male connector having a cavity and a female connector having a cavity, to which another male connector similar to the male connector can be connected,
wherein the connector includes:
a male lock section; and
a female lock section, to which another male lock section similar to the male lock section can be coupled; and
when the connector is in a locked state, in which the female lock section and the other male lock section are coupled, the female connector and the other male connector are connected so that the cavities thereof communicate with each other enabling a liquid to flow therethrough.

The above configuration ensures that the connector can be placed in a locked state easily and assuredly for enabling a liquid to flow.

In the connector of the present invention, preferably, when the female connector and the other male connector are disposed closely to each other, the female lock section and the other male lock section are set in a half-locked state.

This enables an easy transition from the half-locked state to the locked state.

In the connector of the present invention, preferably, when the locked state is released, the flow of liquid is intercepted.

This makes it possible to securely prevent leakage of the liquid.

In the connector of the present invention, preferably, the male lock section has a claw part provided with a male-side engaging part, and an energizing part for energizing the claw part;
the female lock section has a female-side engaging part, which is engageable with a male-side engaging part of the other male lock section similar to the male lock section; and
the connector is set in the locked state through engagement of the female-side engaging part with the male-side engaging part of the other male lock section, and the locked state is released when the male-side engaging part of the other male lock section is pushed against an energizing force of the energizing part.

This makes it possible to place the connector in a locked state more easily and assuredly.

In the connector of the present invention, preferably, the male lock section has a pair of claw parts which are provided with a first male-side engaging part and which can be moved toward and away from each other, and an energizing part for energizing the claw parts away from each other;
the female lock section has a first female-side engaging part, which is engageable with a first male-side engaging part of the other male lock section similar to the male lock section, and an operating part which is operable so as to bring claw parts of the other male lock section closer to each other when the first female-side engaging part and the first male-side engaging part of the other male lock section engage with each other; and
the connector is set in the locked state through engagement of the first female-side engaging part with the first male-side engaging part of the other male lock section.

This makes it possible to place the connector in a locked state more easily and assuredly.

In the connector of the present invention, preferably, the claw parts include a second male-side engaging part;
the female lock section includes a second female-side engaging part; and
a half-locked state, wherein the female connector and the other male connector are located close to each other, is obtained by engagement of the second female-side engaging part with a second male-side engaging part of the other male lock section.

This enables an easy transition from the half-locked state to the locked state.

In the connector of the present invention, preferably, the male lock section includes a tubular ring member, which is disposed on an outer peripheral side of the male connector, so as to be rotatable concentrically with the male connector, and which is provided with a screw thread on an inner peripheral part;
the female connector includes, on an outer peripheral part thereof, a projection that engages with a screw thread of the other male lock section similar to the male lock section; and
the connector is set in the locked state through engagement of the projection with the screw thread of the other male lock section.

This makes it possible to place the connector in a locked state more easily and assuredly.

In the connector of the present invention, preferably, the male connector and the male lock section are disposed adjacent to each other, such that projecting directions thereof are parallel to each other; and
the female connector and the female lock section are disposed adjacent to each other, such that projecting directions thereof are parallel to each other.

This configuration ensures, by checking the female connector and the other male connector, that the connectors made up of the female connector and the other male connector are connected to each other accurately (assuredly). Further, by checking the female lock section and the other male lock section, the configuration ensures that the connectors made up of the female lock section and the other male lock section are connected to each other accurately (assuredly).

In the connector of the present invention, preferably, the connector includes a plurality of female connectors; and
at least one of the female connectors and the male connector are disposed such that the center lines thereof are substantially orthogonal to each other.

This makes it possible to switch the flow direction(s) of the liquid(s) flowing through the connector.

In the connector of the present invention, preferably, the connector includes a plurality of female connectors; and
at least one of the female connectors and the male connector are disposed such that the center lines thereof are parallel to each other, and wherein an opening part of the female connector and an opening part of the male connector are oriented oppositely to each other.

This ensures that, for example, when the two connectors are connected to each other, that the connectors are connected substantially rectilinearly, by connecting the male connector on one side and the female connector on the other side.

In the connector of the present invention, preferably, the connector has a plurality of female connectors, wherein opening parts of the female connectors open in different directions.

This makes it possible to switch the flow direction(s) of the liquid(s) flowing through the connector.

The connector of the present invention, preferably, further includes a seal member formed from an elastic material, which keeps a liquid-tight connection between the female connector and the other male connector when the connector is in a locked state.

This ensures that, in the locked state, the female connector and the other male connector are assuredly connected in a liquid-tight manner.

In the connector of the present invention, preferably, the seal member is disposed in a cavity of the female connector, the seal member having a surface in close contact with an end portion of the other male connector when the connector is in the locked state, and a slit formed in the surface and which is open when the connector is in a locked state.

This ensures that, in the locked state, the female connector and the other male connector are more assuredly connected in a liquid-tight manner.

In the connector of the present invention, preferably, the seal member is disposed in an opening part of the female connector, the seal member having a convergent section that converges while projecting toward the cavity of the female connector.

This ensures that, in the locked state, the female connector and the other male connector are more assuredly connected in a liquid-tight manner.

In the connector of the present invention, preferably, the seal member is annular in shape, the seal member being disposed on an inner peripheral surface of the cavity of the female connector and along a circumferential direction of the cavity.

This ensures that, in the locked state, the female connector and the other male connector are more assuredly connected in a liquid-tight manner.

In the connector of the present invention, preferably, the seal member is annular in shape, the seal member being disposed on the outer peripheral side of the male connector and along a circumferential direction of the male connector.

This ensures that, in the locked state, the female connector and the other male connector are more assuredly connected in a liquid-tight manner.

The connector of the present invention, preferably, includes male connector(s) and female connector(s) totaling three or more in number, and further includes a passage switching means for selecting an arbitrary two cavities from among the cavities of the male and female connectors, and switching liquid passages therein such that the selected two cavities communicate with each other.

This makes it possible to mix a plurality of different kinds of liquids, differing, for example, in liquid composition, at an arbitrary timing.

In the connector of the present invention, preferably, the passage switching means includes a cock, which is turnable relative to the male and female connectors, and which is formed with passages therein corresponding respectively to the cavities, wherein opening and closing of the cavities is selected by turning the cock.

This makes it possible to mix a plurality of different kinds of liquids, differing, for example, in liquid composition, at an arbitrary timing.

In addition, in order to attain the above objects, the present invention also provides:
a connector including a male connector having a cavity, and to which a female connector having a cavity can be connected,
wherein the connector has a male lock section or a female lock section, to which another male lock section similar to the male lock section can be coupled; and
when the connector is in a locked state in which the female lock section and the other male lock section are coupled, or when the connector is in a locked state in which the male lock section and another female lock section similar to the male lock section are coupled, the male connector and the female connector are connected such that the cavities thereof communicate with each other so as to enable a liquid to flow therethrough.

This configuration ensures that the connector can be set in a locked state easily and assuredly, and that, in the locked state, liquid can flow through the connector securely.

Further, in order to attain the above objects, the present invention also provides:
a connector including a female connector having a cavity, and to which a male connector having a cavity can be connected,
wherein the connector has a male lock section or a female lock section, to which another male lock section similar to the male lock section can be coupled; and
when the connector is in a locked state in which the female lock section and the other male lock section are coupled to each other, or when the connector is in a locked state in which the male lock section and another female lock section similar to the male lock section are coupled, the female connector and the male connector are connected such that the cavities thereof communicate with each other so as to enable a liquid to flow therethrough.

This configuration ensures that the connector can be set in a locked state easily and assuredly, and that, in the locked state, liquid can flow through the connector securely.

In addition, in order to attain the above objects, the present invention also provides,
a tube assembly including:
a flexible tube; and
a connector as set forth in any of claims 1 to 20 connected to at least one end of the tube.

This configuration ensures that the connector can be set in a locked state easily and assuredly, and that, in the locked state, liquid can flow through the connector securely.

Further, in order to attain the above objects, the present invention provides,
an infusion tube set including:
at least one connector as set forth in any of claims 1 to 20;
a tube assembly which forms a passage for an infusion, one end of which is connectable to the connector;
a connection section provided at the other end of the tube assembly, and which is connected to a side of a containing section containing the infusion; and
an infusion dosing section including an infusion dosing section side connector connectable to the male connector or to the female connector of the connector, and which is operative to dose a patient with the infusion.

This configuration makes it possible to set the connector in a locked state easily and assuredly, and that, in the locked state, liquid can flow through the connector securely.

In addition, in order to attain the above objects, the present invention provides:
a container for medical use, including a flexible container body in which a liquid can be contained, and a connector as set forth in any of claims 1 to 20 fixed to the container body,
wherein the connector forms a port through which said liquid is injected into and/or discharged from the container body.

This configuration ensures that the connector can be set in a locked state easily and assuredly, and that, in the locked state, liquid can flow through the connector securely.

### BRIEF DESCRIPTION OF THE DRAWINGS

FIG. 1 is a perspective view showing a first embodiment of a connector according to the present invention.
FIG. 2 is a perspective view showing the first embodiment of the connector according to the present invention.
FIG. 3 is a perspective view showing the first embodiment of the connector according to the present invention.
FIG. 4 is a sectional view taken along line A-A of FIG. 1.
FIG. 5 is a sectional view taken along line A-A of FIG. 1.
FIG. 6 is a sectional view taken along line B-B of FIG. 2.
FIG. 7 is a perspective view of a valve element (seal member) included with the connector shown in FIG. 1 (as well as FIGS. 2 and 3).
FIG. 8 is a sectional view taken along line C-C of FIG. 1.
FIG. 9 is a sectional view taken along line D-D of FIG. 3.
FIG. 10 is a longitudinal sectional view showing a second embodiment of the connector according to the present invention.
FIG. 11 is a longitudinal sectional view showing the second embodiment of the connector according to the present invention.
FIG. 12 is a longitudinal sectional view showing a third embodiment of the connector according to the present invention.
FIG. 13 is a longitudinal sectional view showing the third embodiment of the connector according to the present invention.
FIG. 14 is a longitudinal sectional view showing a fourth embodiment of the connector according to the present invention.
FIG. 15 is a longitudinal sectional view showing the fourth embodiment of the connector according to the present invention.
FIG. 16 is a perspective view showing a fifth embodiment of the connector according to the present invention.
FIG. 17 is a perspective view showing a sixth embodiment of the connector according to the present invention.
FIG. 18 is a sectional view taken along line E-E of FIG. 17.
FIG. 19 is a sectional view taken along line F-F of FIG. 17.
FIG. 20 is a perspective view showing a seventh embodiment of the connector according to the present invention.
FIG. 21 is a side view showing a passage switching pattern implemented in the connector shown in FIG. 20.
FIG. 22 is a side view showing a passage switching pattern implemented in the connector shown in FIG. 20.
FIG. 23 is a side view showing a passage switching pattern implemented in the connector shown in FIG. 20.
FIG. 24 is a side view showing a passage switching pattern implemented in the connector shown in FIG. 20.
FIG. 25 is a perspective view showing an eighth embodiment of the connector according to the present invention.
FIG. 26 is a side view showing a ninth embodiment of the connector according to the present invention.
FIG. 27 is a plan view of an infusion tube set according to the present invention.
FIG. 28 is a plan view showing a condition in which infusion tubes in the infusion tube set shown in FIG. 27 are connected.
FIG. 29 is a perspective view of two connectors according to the present invention, which are connected, in the infusion tube set shown in FIG. 28.
FIG. 30 is a partial longitudinal sectional view of a tube assembly according to the present invention.
FIG. 31 is a perspective view of a container for medical use according to the present invention.
FIG. 32 is a perspective view of a container for medical use according to the present invention.

### BEST MODE FOR CARRYING OUT THE INVENTION

Next, the connector, the tube assembly, the infusion tube set and the container for medical use, according to embodiments of the present invention, shall be described based on the preferred embodiments shown in the accompanying drawings.

### <First Embodiment>

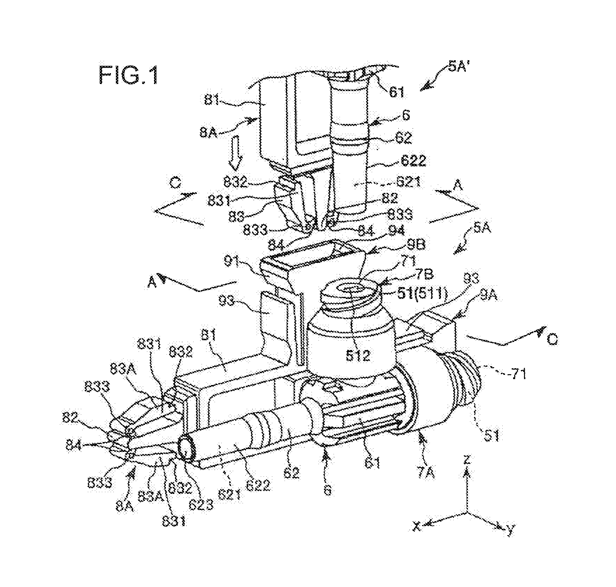

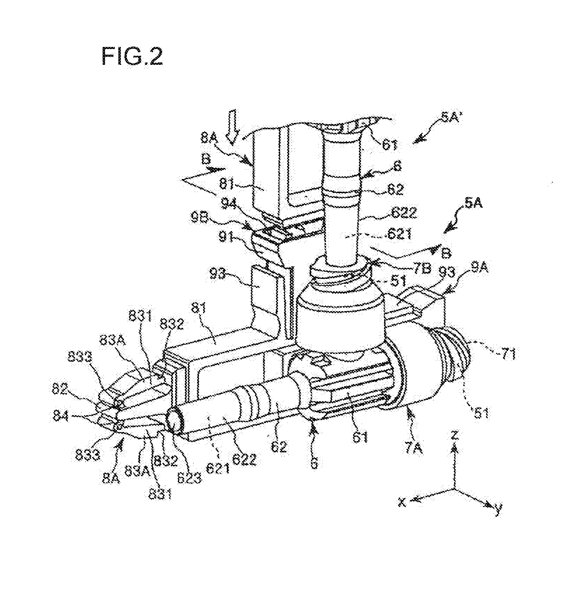

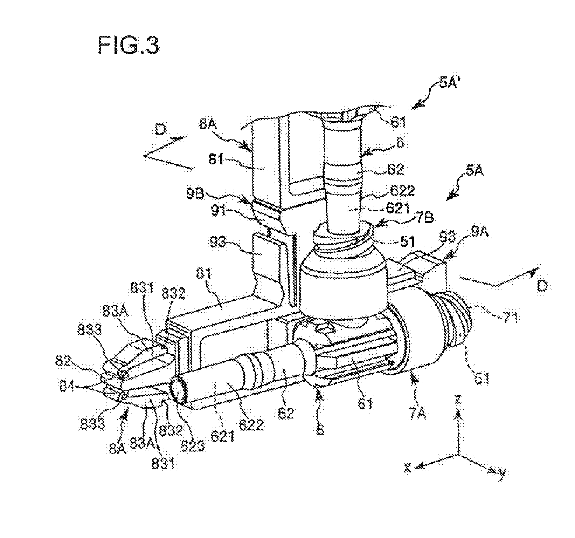

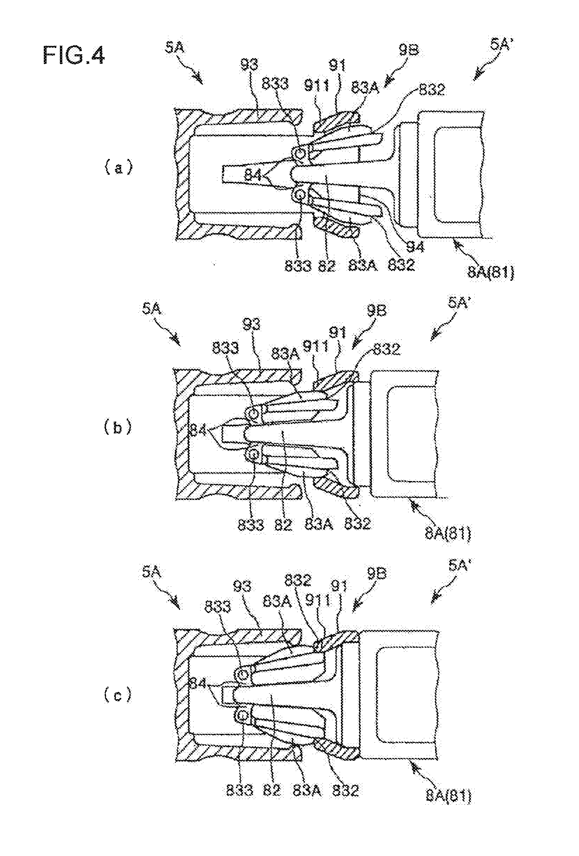

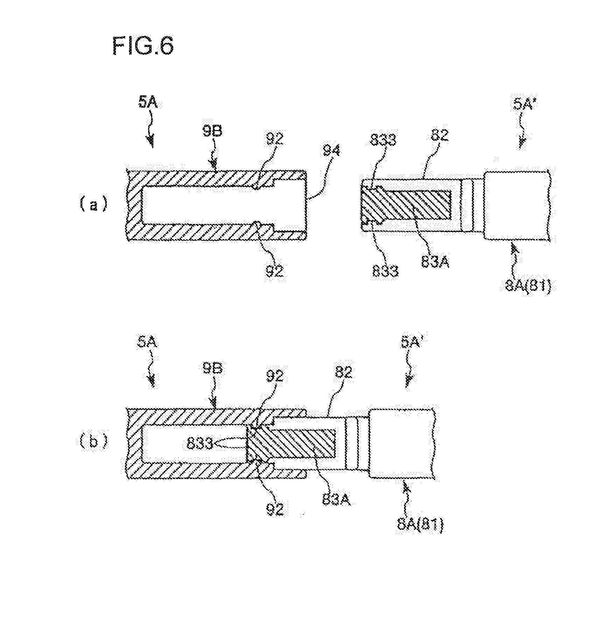

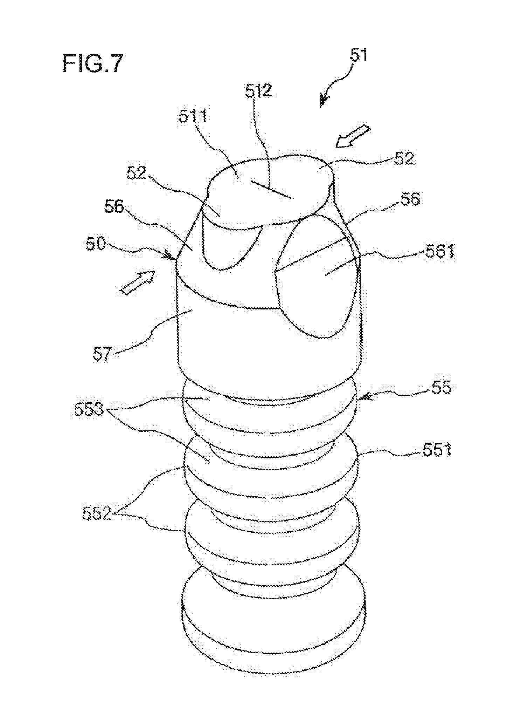

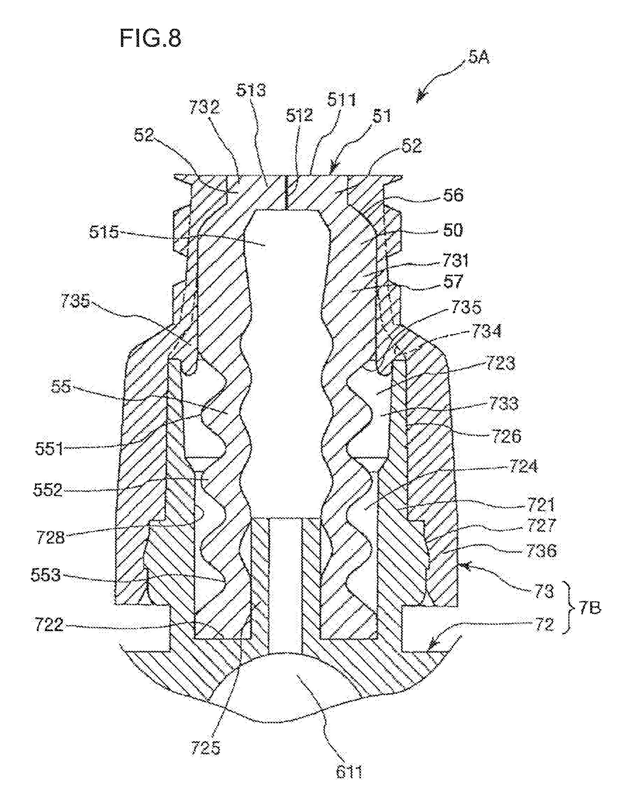

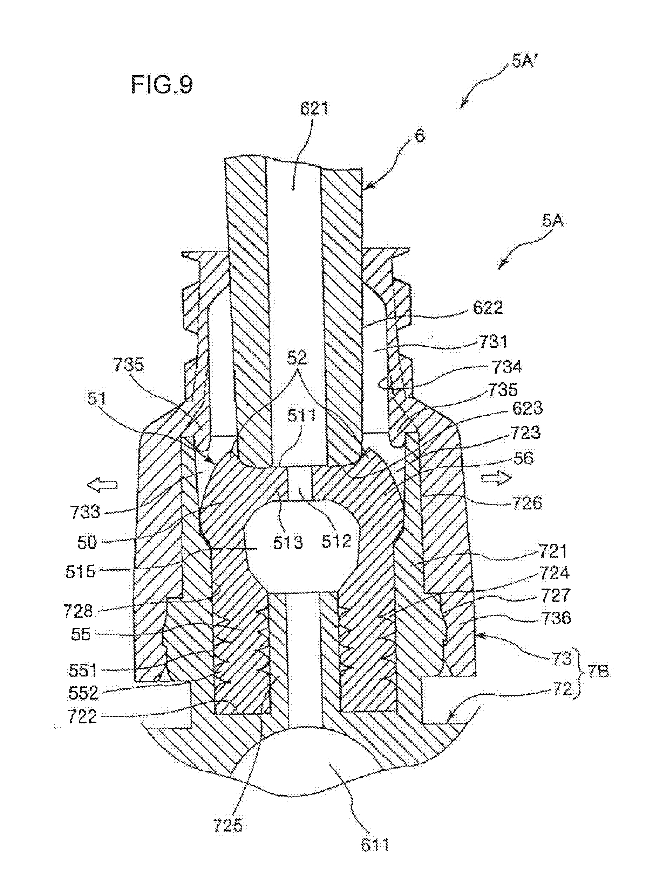

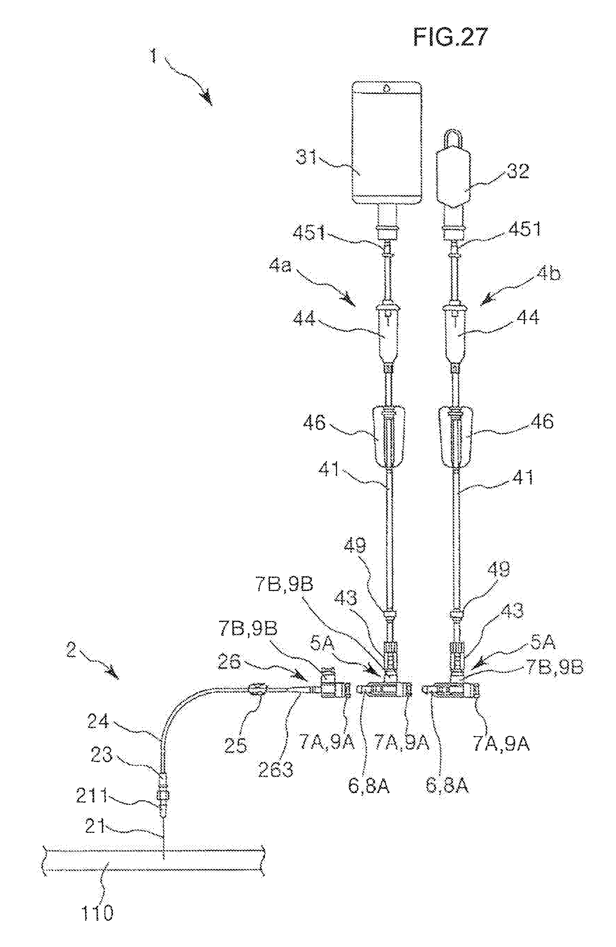

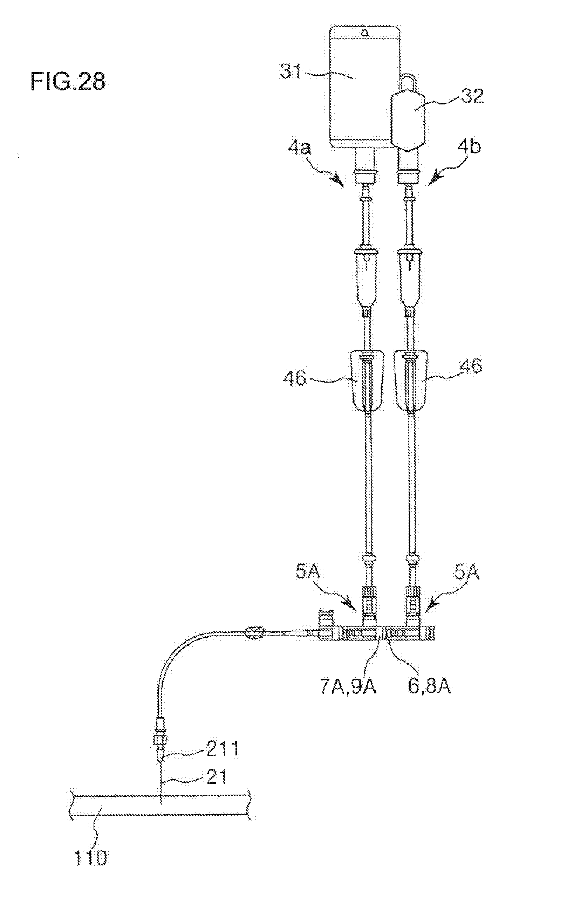

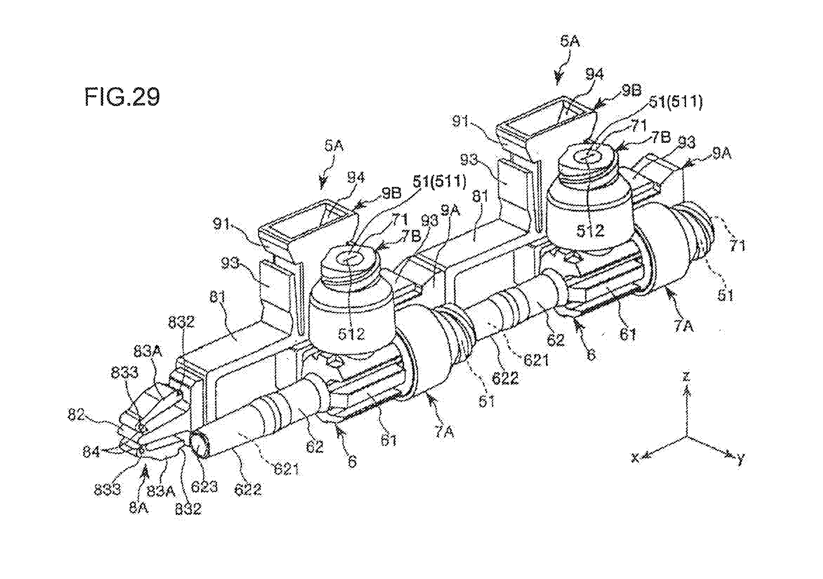

FIGS. 1 to 3 are perspective views showing a first embodiment of the connector according to the present invention; FIGS. 4 to 5 are sectional views taken along line A-A of FIG. 1; FIG. 6 is a sectional view taken along line B-B of FIG. 2; FIG. 7 is a perspective view of a valve element (seal member) included with the connector shown in FIG. 1 (as well as FIGS. 2 and 3); FIG. 8 is a sectional view taken along line C-C of FIG. 1; FIG. 9 is a sectional view taken along line D-D of FIG. 3; FIG. 27 is a plan view of an infusion tube set according to the present invention; FIG. 28 is a plan view showing a condition in which infusion tubes of the infusion tube set shown in FIG. 27 are connected; and FIG. 29 is a perspective view of two connectors according to the present invention, which are connected, in the infusion tube set shown in FIG. 28.

Incidentally, to facilitate the following description, the longitudinal direction of the connector shall be referred to as an "x-axis direction", while directions perpendicular to the x-direction shall be referred to, respectively, as a "y-axis direction" and a "z-axis direction". In addition, in FIGS. 7 to 9, 27 and 28, the lower side shall be referred to as "the tip", and the upper side as "the base end".

The infusion tube set (infusion set) 1 shown in FIGS. 27 and 28 makes up an apparatus (set) for feeding (dosing) an infusion to a living organism (patient).

The infusion includes all types of liquids with which an organism can be dosed, such as a liquid medicine, a correcting electrolyte, physiological saline, etc.

In addition, the drug in the liquid medicine is not particularly limited, wherein examples thereof include sedatives, intravenous anesthetics, anesthetic analgesics, local anesthetics, nondepolarizing muscle relaxants, vasopressors, depressors, coronary vasodilators, diuretics, antiarrhythmic agents, bronchodilators, styptics, vitamin agents, antibiotic agents, and/or lipid emulsions, etc.

As shown in FIG. 27, the infusion tube set 1 includes a first infusion tube (first tube assembly (tube assembly)) 4b, a second infusion tube (second tube assembly (tube assembly)) 4a, connectors 5A to which side ends of the first infusion tube 4b and the second infusion tube 4a can be connected, spike needles 451 (connection sections) provided at other side ends of the first infusion tube 4b and the second infusion tube 4a, and an infusion dosing section 2 for dosing a patient with the infusion(s). These components shall be described below in turn.

As shown in FIGS. 1 to 3, the connector 5A includes a male connector (male-formed connector) 6, female connectors (female-formed connectors) 7A and 7B, a male lock section (male-formed lock section) 8A, female lock sections (female-formed lock sections) 9A and 9B, and a valve element (seal member) 51. Incidentally, FIGS. 1 to 3 illustrate, respectively, a condition before the connector 5A is connected to another connector 5A', a half-locked state of the connector 5A and the other connector 5A', and a locked state between the connector 5A and the other connector 5A'.

Herein, the term "connector 5A'" implies a connector having a male connector 6 and a male lock section 8A, similar to those of the connector 5A.

In addition, the term "half-locked (tentatively fixed) state" implies a state in which the connector 5A and the connector 5A' are not fully connected, i.e., a condition in which the connector 5A and the other connector 5A' can be easily separated from each other.

Further, the term "locked state" implies a state in which the connector 5A (female lock section 9B) and the connector 5A' (male lock section 8A) are fully connected, i.e., a condition in which the connection between the connector 5A and the connector 5A' cannot easily be released, unless an operating part 93 of the female lock section 9B of the connector 5A is operated, as shall be described later.

As shown in FIG. 1, the male connector 6 includes a male connector body 61 and a tubular section 62.

The tubular section 62 projects in the x-axis positive direction from the male connector body 61. The tubular section 62 has a liquid passage (cavity) 621 therein through which a liquid passes, and a lure tapered part 622.

The liquid passage 621 communicates with the inside of the male connector body 61.

The lure tapered part 622 is formed on an outer peripheral portion of the opening part 623 of the male connector 6, such that the outside diameter of the tapered part 622 gradually decreases toward the direction of the opening part 623 (i.e., in the x-axis positive direction).

The female connectors 7A and 7B form portions to which the male connector 6 of the connector 5A' can be connected. The female connector 7A and the female connector 7B have substantially the same shape (configuration). Taking this into account, only the female connector 7B shall be described below, as representative of both female connectors 7A and 7B.

As shown in FIG. 8, the female connector 7B includes a female connector body 72 and a cap section (cap) 73.

The female connector body 72 shown in FIG. 8 includes a valve element disposing section 721, which is in the shape of a bottomed tube. The valve element disposing section 721 is formed therein with a second cavity (cavity) 723 on the base end side, and a third cavity (cavity) 724 on the tip side, which communicates with the second cavity 723. The second cavity 723 has an inside diameter larger than that of a first cavity (cavity) 731, which is formed in the cap section 73 as described later, whereas the third cavity 724 (inner peripheral surface 728) has an inside diameter smaller than that of the second cavity 723. The inside diameter of the third cavity 724, preferably, is slightly greater than the maximum outside diameter of a barrel part 55 (outer peripheral surface 551) of the valve element 51, which shall be described later.

In addition, an inside projection 725 composed of a tubular body is provided at a central portion of a bottom surface 722 of the valve element disposing section 721 (female connector body 72). When the male connector 6 is connected to the first cavity 731 (connection port 732) and the valve element 51 begins to be pressed, the interior (inside) of the valve element 51 is supported by the inside projection 725, such that buckling of the valve element 51 (i.e., bending of the valve element 51 in a U-shape) can be prevented (see FIG. 9). Further, liquid passing through the connector 5A can be prevented from becoming stagnant therein.

In addition, the cavity of the inside projection 725 communicates with a passage 611, which is formed inside of the male connector body 61 and through which liquid can pass. This ensures that the second cavity 723 and the third cavity 724 communicate, via the inside projection 725, with the passage 611.

In addition, a stepped part 727, which is greater in diameter than the base end side, is provided on the tip side of an outer peripheral surface 726 of the valve element disposing section 721.

The cap section 73, as shown in FIG. 8, is provided therein with a space (cavity) containing the valve element 51, and is coupled to the valve element disposing section 721 of the female connector body 72.

The cap section 73 includes therein a first cavity 731, in which a head part 50 of the valve element 51, to be described later, can be inserted. The cap section 73 also includes a fitting section 733, which communicates with the first cavity 731, and which is greater in diameter than the first cavity 731.

The first cavity 731 has a shape corresponding to the outer shape of the head part 50 of the valve element 51. In addition, the connection port (connection section) 732 that connects to the male connector 6 is formed on the base end side of the first cavity 731, wherein a diameter thereof is smaller than the diameter on the tip side of the first cavity 731.

An inner peripheral surface 734 of the first cavity 731 is provided with a plurality of ribs 735, which project in a radial direction of the first cavity 731, and which are arranged at regular intervals along the circumferential direction of the inner peripheral surface 734. When the male connector 6 is connected to the connector 5A (connection port 732), the valve element 51 is supported by the ribs 735, such that buckling of the valve element 51 (i.e., dropping of the valve element 51) can be prevented. The number of ribs 735 is not particularly limited, for example, the number may preferably be from two to ten, and more preferably, from four to eight.

A stepped part 736, which fits with the stepped part 727 of the valve element disposing section 721, is formed on the tip side of the fitting section 733, wherein the diameter thereof is greater than the diameter on the base end side of the fitting section 733. Preferably, the inside diameter of the stepped part 736 is approximately equal to or slightly smaller than the outside diameter of the stepped part 727 of the valve element disposing section 721. This enables a firm fitting (coupling) (liquid-tight contact) to be achieved between the cap section 73 (stepped part 736) and the female connector body 72 (stepped part 727), and therefore makes it possible to prevent the liquid inside the connector 5A from leaking. In addition, when the cap section 73 and the female connector body 72 are coupled, the first cavity 731 and the second cavity 723 communicate with each other, wherein the valve element 51 is disposed (contained) within the space composed of the first cavity 731, the second cavity 723, and the third cavity 724.

Incidentally, the method of fixing the female connector body 72 and the cap section 73 is not limited to the aforementioned fitting. For example, the female connector body 72 and the cap section 73 may be fixed to each other by caulking, adhesion with an adhesive, or by fusing, such as heat fusing, ultrasonic fusing, or the like.

As shown in FIG. 1 (as well as FIGS. 2 and 3), the female connector 7A is disposed in the x-axis negative direction in relation to the male connector body 61 (male connector 6). Specifically, the female connector 7A is disposed such that a center line thereof and a center line of the male connector 6 are parallel to each other, wherein the opening part 71 of the female connector 7A and the opening part 623 of the male connector 6 are oriented in opposite directions.

This ensures, for example, when the two connectors 5 are connected to each other, that the connectors are connected substantially rectilinearly, by connecting the male connector 6 of one side with the female connector 7A of the other side.

The female connector 7B is disposed in the z-axis positive direction in relation to the male connector body 61 (male connector 6). In other words, the female connector 7B is disposed such that a center line thereof is substantially orthogonal to the center line of the male connector 6.

By providing the female connectors 7A and 7B in this manner, liquid can be supplied to the male connector 6 from two different directions. Also, liquid from the male connector 6 can be bifurcated in two different directions.

In addition, for example, when the male connector 6 of the connector 5A' is connected to the female connector 7B, the lure tapered part 622 of the connector 5A' is fitted securely to an edge part (inner peripheral part) of the connection port 732 of the female connector 7B, i.e., the male connector 6 of the connector 5A' is connected in a liquid-tight manner to the female connector 7B (see FIG. 9).

As shown in FIGS. 8 and 9, the valve element 51 is contained (fixed) within each of the female connectors 7A and 7B.

The valve elements 51 are formed of an elastic material. Examples of suitable elastic materials include various rubber materials such as natural rubber, isoprene rubber, butadiene rubber, styrene-butadiene rubber, nitrile rubber, chloroprene rubber, butyl rubber, acrylic rubber, ethylenepropylene rubber, hydrin rubber, urethane rubber, silicone rubber, fluororubber, etc., and various thermoplastic elastomers based on styrene, polyolefin, polyvinyl chloride, polyurethane, polyester, polyamide, polybutadiene, transpolyisoprene, fluororubber, chlorinated polyethylene, or the like. Such elastic materials may be used either singly or in a combination of two or more. The use of such elastic materials makes it possible to provide appropriate elasticity at a top face 511 of the valve element 51, thereby facilitating the top face 511 to be brought into close contact with the opening part (end part) 623 of the male connector 6 (see FIG. 9).

As shown in FIG. 7, the valve element 51 includes the head part 50 as well as the barrel part 55, which is provided (formed) on the tip side of the head part 50.

As shown in FIG. 8, the head part 50 is in the shape of a bottomed tube, and is formed with a cavity 515 therein through which liquid passes. Further, the head part 50 includes a slit 512 penetrating from the flat top face 511 (bottom part 513) to the cavity 515. The slit 512 is substantially in the shape of a straight line segment, wherein the simple shape of the slit 512 enables the slit 512 to be opened more easily (assuredly). In addition, the flat surface shape of the top face 511 enables the top face 511 (and the slit 512) to be easily disinfected.

As shown in FIG. 1, the top face 511 is exposed from the opening part 71 of the female connector 7A. Further, the top face 511 is located substantially in the same plane as the end face of the female connector 7A.

The head part 50 has a tapered part 56 provided near the top face 511, which is tapered such that the outside diameter thereof gradually increases along the direction toward the barrel part 55 (i.e., in the z-axis negative direction). The head part 50 also has a fixed-outside-diameter part 57, provided at the tip of the tapered part 56.

In addition, the tapered part 56 is provided with a cutout part (deficiency) 561 formed by removing a portion of the tapered part 56. More specifically, the head part 50 is provided with the cutout part 561 therein, which is formed by removing a portion ranging from the tapered part 56 to the fixed-outside-diameter part 57.

This ensures that when the male connector 6, which is connected to (inserted into) the connector 5A, is withdrawn, i.e., when the locked state is released, the valve element 51 (head part 50) more easily enters into the first cavity 731 of the cap section 73, and therefore, that the slit 512 can be closed more securely.

Further, the head part 50 is provided with two projecting abutment parts 52, which are pressed when the slit 512 is closed. The two abutment parts 52 are formed near the top face 511 of the head part 50, and project in directions opposite to the directions (directions of the arrows shown in FIG. 7) in which the slit 512 is closed.

The abutment parts 52 ensure that when the head part 50 is inserted into the first cavity 731 of the cap section 73, the inner peripheral surface 734 of the first cavity 731 presses against the abutment parts 52, so that the slit 512 can be closed more securely (see FIG. 8). In addition, this makes it possible to enhance pressure resistance of the valve element 51 (slit 512), with respect to the pressure (inside pressure) of the liquid that is present inside the connector 5A.

When the male connector 6 is not connected, the head part 50, configured as described above, is inserted into the first cavity 731 of the cap section 73, with the slit 512 being closed (see FIG. 8).

As shown in FIG. 7, the barrel part 55 is composed of a bellows-like tubular body. Specifically, the barrel part 55 has a bellows-like outer shape, in which large-diameter ring parts 552 and small-diameter ring parts 553 are alternately arranged in the axial direction. The barrel part 55 thus configured functions as a deforming part (energizing means), which acts to energize the valve element 51 from the tip side toward the base end side (i.e., in the direction in which the head part 50 is inserted into the first cavity 731 of the cap section 73).

Insofar as the barrel part 55 functions as a deforming part, it is unnecessary to provide the connector 5A separately with a component part constituting an energizing means. Accordingly, the number of component parts making up the connector can be reduced and simplified in structure.

In addition, while the barrel part 55 bears most of the restoring force, by which the valve element 51 is restored from the tip side toward the base end side, the head part 50 may also bear a portion of the restoring force.

Further, in the barrel part 55 (tubular body), the number of projected portions (large-diameter ring parts 552) making up the bellows-like shape is three or four. In other words, the number of recessed portions (small-diameter ring parts 553) making up the bellows-like shape is four or five.

In addition, although in the configuration shown in FIG. 9 the slit 512 is opened by being pressed by the male connector 6, the invention is not limited to such a configuration. For example, the slit 512 may be opened as a result of being pierced by the male connector 6, or may be opened as a result of being pierced by a needle or pin disposed inside of the valve element 51 (inside the female connector 7B).

As shown in FIG. 1, the male lock section 8A includes a base (male lock body) 81, a projecting part 82 projecting from the base 81, a pair of claw parts 83A, 83A provided at the projecting part 82, and energizing parts 84 for energizing the claw parts 83A.

The base 81 has an elongate shape.

The male connector body 61 of the male connector 6 is firmly attached (fixed) in the y-axis positive direction of the base 81 (male lock section 8A), adjacently to the base 81. In addition, the projecting part 82 is formed so as to project in the x-axis positive direction of the base 81.

The claw parts 83A, 83A are provided, respectively, in the z-directions (upward and downward directions as shown in FIG. 1) of the projecting part 82. Respective ends 831 of the claw parts 83A, 83A are moved toward and away from each other along the z-axis direction.

Each of the claw parts 83A is provided, on its end 831, with a first male-side engaging part 832, formed by cutting off (removing) a portion of the end 831.

In addition, both side surfaces of the claw parts 83A are each provided with a recessed second male-side engaging part 833, which faces in the y-axis direction.

The depth of the second male-side engaging part 833 is not particularly limited. For example, the depth is preferably 0.05 to 0.6 mm, and more preferably, 0.1 to 0.4 mm.

The energizing parts 84 are disposed between the projecting part 82 and the claw part 83A. The energizing parts 84 energize the claw parts 83A, 83A such that the ends 831 (first male-side engaging parts 832) of the claw parts 83A are spaced away from each other.

The male lock section 8A, which is configured in this manner, is disposed at a position corresponding to (adjacent to) the male connector 6 (see FIG. 1). In addition, the male lock section 8A and the male connector 6 are formed along the x-axis direction, i.e., the connecting directions (projecting directions) thereof are formed parallel to each other.

As shown in FIG. 1, the female lock sections 9A and 9B, respectively, make up portions to which the male lock section 8A of the connector 5A' can be coupled.

The female lock section 9A is disposed in the x-axis negative direction in relation to the base 81 (male lock section 8A). Further, the female lock section 9B is disposed in the z-axis positive direction in relation to the base 81 (male lock section 8A). The female lock sections 9A and 9B are substantially the same in shape (configuration), and therefore, only the female lock section 9B shall be described below, as representative of both female lock sections 9A and 9B.

The female lock section 9B is roughly tubular in overall shape.

As shown in FIGS. 4 and 6, the female lock section 9B includes a first female-side engaging part 91, which is capable of engagement with the first male-side engaging part 832 of the male lock section 8A of the connector 5A', a second female-side engaging part 92, which is capable of engagement with the second male-side engaging part 833 of the male lock section 8A of the connector 5A', and an operating part 93 serving to operate the claw parts 83A of the male lock section 8A of the connector 5A'.

As shown in FIG. 4, the first female-side engaging part 91 is formed such that the size of an inner peripheral part thereof, on a side of the opening part 94 of the female lock section 9B, gradually decreases along the z-axis negative direction (i.e., the leftward direction in FIG. 4). As shown in FIG. 4(c), when an end part 911 of the first female-side engaging part 91 engages with the first male-side engaging parts 832 of the male lock section 8A of the connector 5A', the connector 5A (female lock section 9B) and the connector 5A' (male lock section 8A) are placed in a locked state.

As shown in FIG. 6, the second female-side engaging parts 92 are disposed so as to project at intermediate portions on the inner peripheral part of the female lock section 9B. As shown in FIG. 6(b), when the second female-side engaging parts 92 engage with the second male-side engaging parts 833 of the male lock section 8A of the connector 5A', the connector 5A and the connector 5A' are placed in a half-locked state.

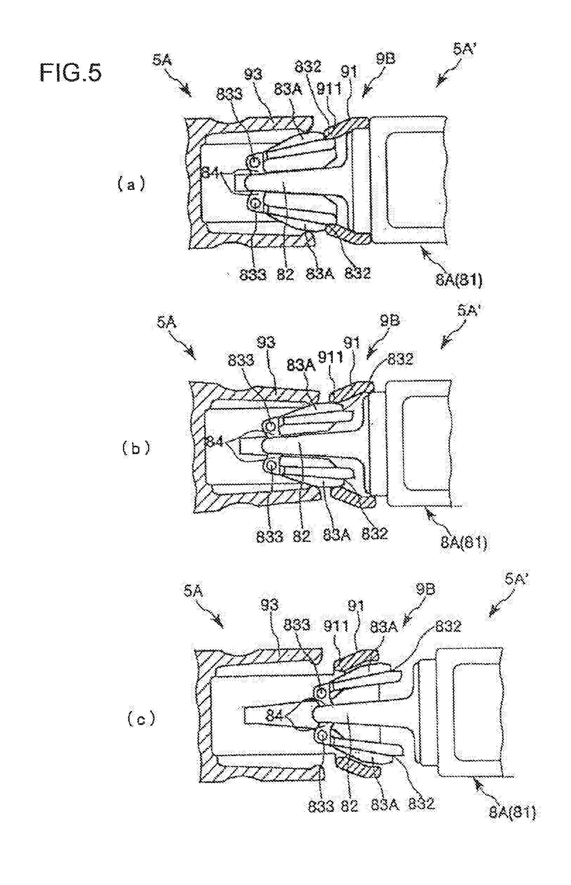

As shown in FIG. 5, the operating part 93 is composed of a small piece, which is disposed in the z-axis negative direction in relation to the first female-side engaging part 91. The operating part 93 operates (presses) the claw parts 83A of the connector 5A' (male lock section 8A) in the locked state, so as to urge the end parts 831 of the claw parts 83A toward each other.

In addition, the operating part 93 is formed such that an outside surface thereof is substantially coincident with the outside surface of the first female-side engaging part 91. This makes it possible to prevent a user from unintentionally touching (pressing) the operating part 93. That is, the locked state can be prevented from being released unintentionally.

The female lock section 9B, configured as described above, is located at a position corresponding to (adjacent to) the female connector 7B (see FIG. 1). In addition, the female lock section 9B and the female connector 7B are both formed along the z-axis direction, i.e., such that the directions (formation direction) in which they are connected are oriented parallel to each other.

Thus, in the connector 5A, the male components (the male lock section 8A and the male connector 6) correspond to each other, and the female components (the female lock section 9B and the female connector 7B) correspond to each other. This makes it possible to correctly (securely) interconnect the connector 5A and the connector 5A', by checking the female connector 7B and the male connector 6, and also to correctly (securely) interconnect the connector 5A and the connector 5A', by checking the female lock section 9B and the male lock section 8A.

Incidentally, the number of second male-side engaging parts 833 is not limited to four. For example, the number may be any other even number, such as two or six.

In addition, the depth at which the second female-side engaging parts 92 engage with the second male-side engaging parts 833 in the half-locked state is not particularly limited. For example, such a depth is preferably 0.02 to 0.5 mm, and more preferably, 0.15 to 0.35 mm. This makes it possible to appropriately set the engaging force at which the second female-side engaging parts 92 engage with the second male-side engaging parts 833.

The materials constituting portions of the connector 5A, other than the valve element 51, are not particularly limited. Examples of materials that can be used include various resin materials such as polyolefins including polyethylene, polypropylene, polybutadiene, etc., polyurethanes, polystyrenes, acrylic resins such as polymethyl methacrylate, etc., polycarbonates, polyamides, polyesters such as polyethylene terephthalate, etc., ABS resins, AS resins, fluororesins such as ionomers, etc., various thermoplastic elastomers based on polyurethane, polyester, polyamide, olefin, styrene or the like, various metallic materials such as stainless steel, aluminum, titanium, etc., and various glass materials. Such materials may be used singly or in any arbitrary combinations thereof (e.g., as composite materials).

Next, a connection process for interconnecting the connector 5A and the connector 5A' (half-locked state, locked state) shall be described below.

Starting from a condition in which the connectors 5A and 5A' are spaced from each other, as shown in FIG. 1 (FIG. 6(a)), the male lock section 8A of the connector 5A' is moved so as to approach the female lock section 9B of the connector 5A.

While the male lock section 8A of the connector 5A' is inserted into the female lock section 9B of the connector 5A, as shown in FIG. 4(a), the first female-side engaging part 91 of the connector 5A presses the claw parts 83A of the connector 5A' against energizing forces exerted by the energizing parts 84 of the connector 5A'.

When the male lock section 8A of the connector 5A' is pushed further into the female lock section 9B of the connector 5A, as shown in FIG. 4(b), the second male-side engaging parts 833 of the connector 5A' engage with the second female-side engaging parts 92 of the connector 5A, as shown in FIG. 6(b). Namely, the connector 5A' and the connector 5A are placed in a half-locked state. In this instance, the male connector 6 (opening part 623) of the connector 5A' and the female connector 7B (top face 511) of the connector 5A are placed in contact with (in proximity to) each other (see FIG. 2). This ensures that, while transitioning from the half-locked state to the locked state, the end face (opening part 623) of the male connector 6 of the connector 5A' can securely and swiftly press against the top face 511 of the valve element 51 of the connector 5A.

When the male lock section 8A of the connector 5A' is pushed further into the female lock section 9B of the connector 5A, the second male-side engaging parts 833 of the connector 5A' and the second female-side engaging parts 92 of the connector 5A become disengaged from each other, i.e., the half-locked state is released.

As the male lock section 8A of the connector 5A' and the female lock section 9B of the connector 5A are brought closer to each other, starting from the condition mentioned immediately above, the first male-side engaging parts 832 of the connector 5A' engage with the first female-side engaging part 91 of the connector 5A, i.e., the connector 5A' and the connector 5A are placed in a locked state (see FIG. 4(c)). Thus, the connector 5A' and the connector 5A can easily be brought from the half-locked state into the locked state.

Incidentally, in the half-locked state, the male connector 8A of the connector 5A' and the valve element 51 of the connector 5A are in proximity to each other. However, in this case, they may also be in close contact with each other.

As shown in FIG. 9, in the locked state, the opening part 623 of the male connector 6 of the connector 5A' presses the top face 511 of the head part 50 of the connector 5A in the axial direction. As a result, the barrel part 55 is deformed (compressed) in the axial direction, wherein the head part 50 moves from the first cavity 731 into the second cavity 723. Although the head part 50 is restricted by the inner peripheral surface 734 of the first cavity 731 when the head part 50 is located inside the first cavity 731, movement of the head part 50 into the second cavity 723 removes or suppresses this restriction on the outer peripheral surface of the head part 50. As a result, the head part 50 can be enlarged in diameter, or deformed sufficiently in the directions of the arrows shown in FIG. 9, due to compression thereof in the axial direction. Therefore, the valve element 51 (slit 512) can be opened sufficiently and securely. Also, this ensures that the liquid passage 621 of the male connector 6 of the connector 5A' communicates with the cavity (hollow portion) of the inside projection 725 of the female connector 7B of the connector 5A, i.e., that they are connected so as to enable a liquid to flow. Accordingly, liquid can pass smoothly therethrough at the time of performing, for example, infusion, blood transfusion, or nutritional dosing.

In addition, in the locked state, the opening part 623 of the male connector 6 of the connector 5A' presses the head part 50 of the valve element 51, against an energizing force of the barrel part 55 of the valve element 51, such that the opening part 623 of the male connector 6 and the top face 511 of the head part 50 (valve element 51) come into close contact with each other. This makes it possible to maintain the connection between the male connector 6 and the female connector 7B, i.e., to assuredly connect them in a liquid-tight manner.

In addition, the locked state of the connector 5A' and the connector 5A can be released by operating the operating part 93 of the connector 5A.

In other words, starting from the locked state shown in FIG. 5(a), the claw parts 83A of the connector 5A' are pressed against the operating part 93 of the connector 5A, as shown in FIG. 5(b). As a result, the first female-side engaging part 91 of the connector 5A and the first male-side engaging parts 832 of the connector 5A' become disengaged from each other (the engagement is released). In this instance, the head part 50 of the valve element 51 moves again into the first cavity 731, as a result of the restoring force of the barrel part 55, so as to close the slit 512 as mentioned above. As a result, flow of liquid between the connector 5A and the connector 5A' is interrupted, such that the liquid can securely be prevented from leaking out from the connector 5A, for example.

Thereafter, as shown in FIG. 5(c), the connector 5A' is pulled outward, thereby spacing the connector 5A' and the connector 5A from each other.

Incidentally, when the connectors 5A and 5A' are disengaged, if a fitting force between the female connector 7B of the connector 5A and the male connector 6 of the connector 5A' is weak, the connector 5A' can be released and spring away from the connector 5A due to the energizing force of the valve element 51. Namely, the connector 5A' can easily be released from the connector 5A.

In addition, when released, the energizing force (elastic force) of the energizing parts 84 of the connector 5A' can be utilized as a repelling force, which is exerted from the claw parts 83A onto the inner peripheral surface of the first female-side engaging part 91 of the connector 5A.

In addition, since the connector 5A' can be released by springing away from the connector 5A, the connector 5A' is repelled from the connector 5A, for example, whenever the connection between the connector 5A' and the connector 5A is loose. This makes it possible to determine whether or not the connectors 5A' and 5A have been placed securely in a locked state.

Further, when the above-mentioned fitting force is strong, the connector 5A' does not become released by springing away from the connector 5A, and therefore, liquid that passes through the connector 5A (e.g., a liquid which should not be scattered into the surrounding environment, such as a carcinostatic) can be prevented or restrained from being scattered into the surrounding environment (to the exterior).

As has been described above, the connector 5A and the connector 5A' can be connected together perfectly, and securely placed in a locked state, through a simple operation, simply by pushing the male lock section 8A of the connector 5A' into the female lock section 9B of the connector 5A.

In addition, substantially at the same time that the locked state is attained, the female connector 7B of the connector 5A and the male connector 6 of the connector 5A' are connected in a liquid-tight manner.

Further, in the half-locked state, deformation of the valve element 51 is restrained (prevented). For example, in the case where an infusion tube set 1 is shipped with the connector 5A and the connector 5A' in the half-locked state, the valve element 51 is prevented from remaining in a deformed state for a long period of time until the infusion tube set 1 is used. Accordingly, a situation in which the valve element 51 undergoes plastic deformation, with the result that the slit 512 remains constantly open, can be avoided.

Although, in the locked state in the configuration shown in the figures, the lure tapered part 622 of the male connector 6 of the connector 5A' is fitted into (i.e., is placed in close contact with) the inner peripheral part of the connection port 732 of the female connector 7B of the connector 5A, the invention is not limited to such a configuration. For example, the lure tapered part 622 does not have to be fitted into the inner peripheral part of the connection port 732. In other words, a gap may be formed between the lure tapered part 622 and the connection port 732. However, when the lure tapered part 622 of the male connector 6 of the connector 5A' is fitted into the inner peripheral part of the connection port 732 of the female connector 7B of the connector 5A, the male connector 6 of the connector 5A' and the female connector 7B of the connector 5A are connected together in a more liquid-tight manner.

In addition, as described above, the connector 5A includes one male connector 6, two female connectors 7A and 7B, one male lock section 8A, and two female lock sections 9A and 9B, however, the invention is not limited to this configuration. For example, the connector 5A may include one male connector 6 and one male lock section 8A or one female lock section 9A (or female lock section 9B, equivalently), or may include one female connector 7A (or female connector 7B, equivalently) and one male lock section 8A or one female lock section 9A (or female lock section 9B, equivalently).

As shown in FIG. 27, the infusion dosing section 2 includes an indwelling needle or catheter (in this embodiment, an indwelling needle 21), which is set so as to indwell within a blood vessel 110 of a patient, and an infusion dosing section side connector 26, connected to a base end part of the indwelling needle or catheter (in this embodiment, the indwelling needle 21).

The indwelling needle 21 (or catheter) may comprise a metallic needle, such as a winged phleboclysis needle, depending on the indwelling site, but is preferably formed from a flexible polymer material, examples of which include thermoplastic resins including polyolefins such as polyethylene, polypropylene, etc., polyesters, polyurethanes, etc.

The infusion dosing section side connector 26 includes a female lock section 9A and a female connector 7A, which can be connected together respectively with a male lock section 8A and a male connector 6 of a connector 5A, which is connected to a second infusion tube 4a, a female lock section 9B and a female connector 7B which are similar to the female lock section 9A and the female connector 7A, and a male connector 263.

In addition, the axis of the male connector 263 and the axis of the female connector 7A coincide substantially with each other, whereas the axis of the female connector 7B is substantially orthogonal to such axes. Namely, the male connector 263 and the female connector 7A are oriented in opposite directions, whereas the female connector 7B is oriented substantially perpendicularly to the male connector 263 and the female connector 7A.

In the infusion dosing section side connector 26, a tube 24 that passes through a stop clamp 25 capable of providing one-stroke clamping is connected in a liquid-tight manner to the male connector 263. A male connector 23 is connected to the tip side of the tube 24. The male connector 23 is not particularly limited, insofar as it can be connected in a liquid-tight manner to the outlet port 211 of an indwelling needle or catheter. Accordingly, the male connector 23 may be a lure connector, a lure lock connector, or the like. Preferably, however, the male connector 23 is a lure lock connector.

The indwelling needle 21 has an outlet port 211 formed at the base end thereof, wherein the male connector 23, which is attached to the infusion dosing section side connector 26, is connected in a liquid-tight manner to the outlet port 211.

Next, the first infusion tube 4a and the second infusion tube 4b shall be described below. Since the first infusion tube 4b and the second infusion tube 4a are the same or equivalent in structure, only the second infusion tube 4a shall be described, as representative of both the first and second infusion tubes 4a, 4b.

The second infusion tube 4a has a tube 41, which is flexible and constitutes a liquid passage, a connector 43 provided at one end (tip) of the tube 41, and the bottle needle 451, which is provided at the other side of the tube 41 (at the other end (base end) in this embodiment), and which has a sharp needle point serving as a connection section connected to one side of an infusion bag (infusion container) (containing section) 31 containing an infusion therein.

Examples of materials that can be used to form the tube 41 include soft polyvinyl chloride, ethylene-vinyl acetate copolymer, polyethylene, polypropylene, polybutadiene, etc., and other materials composed primarily of these materials.

In addition, a slide type forceps (slide forceps) 46 and a dripping cylinder 44 are disposed at intermediate positions in the tube 41, serving as a flow control means for controlling the flow rate of the infusion. The slide forceps 46 is not particularly limited, and conventionally known types, for example, those described in Japanese Laid-Open Patent Publication No. 2004-49319, can be used.

Further, the slide forceps 46 may be replaced by other appliances, such as a roller type forceps, insofar as the flow rate of the infusion can be controlled thereby.

A predetermined infusion is stored in the infusion bag 31. When the bottle needle 451 penetrates (pierces) a stopper (rubber stopper) of the infusion bag 31, the infusion bag 31 and the second infusion tube 4a are connected to each other through the bottle needle 451, resulting in a condition whereby the infusion is supplied to the second infusion tube 4a from the infusion bag 31.

The dripping cylinder 44 is disposed in the vicinity of the bottle needle 451. The dripping cylinder 44 enables the flow rate of the infusion to be checked visually.

In addition, a check valve 49 is disposed between the connector 43 and the dripping cylinder 44. The check valve 49 comprises a one-way valve, which allows flow in one direction from the infusion bag 31 toward the connector 5A. The check valve 49 may be disposed at any position between the connector 43 and the dripping cylinder 44, however, the check valve 49 preferably is disposed near the connector 43, and more preferably, is disposed inside of the connector 43.

The check valve 49 includes a valve body, provided with a pair of plate-like opening/closing members (not shown) on an inside portion thereof. Such opening/closing members are maintained in close contact with each other by elastic (restoring) forces, whereby the passage inside of the check valve 49 is closed thereby. When the infusion flows in one direction from the tip side toward the base end side, the infusion exerts a pressure on outside surfaces of the opening/closing members, such that the opening/closing members are brought into close contact with each other. Accordingly, the infusion does not flow from the tip side toward the base end side.

On the other hand, when the infusion flows in a direction from the base end side toward the tip side, a predetermined pressure is exerted by the infusion on the base end side (tapered surfaces) of the opening/closing members, such that the opening/closing members are displaced away from each other by the pressure, and the passage inside of the check valve 49 opens. This allows the infusion to flow from the base end side toward the tip side.

The check valve 49 may be omitted in cases where injection is secured by an infusion pump or the like. Preferably, however, the check valve 49 is provided. The aforementioned second infusion tube 4a has the following merit. Specifically, even when an infusion is fed from the first infusion tube 4b, which is connected to the connector 5A of the second infusion tube 4a under a certain pressure, the infusion can be prevented by the check valve 49 from flowing upstream (toward the base end side) of the second infusion tube 4a. Thus, dosing of a patient with the infusion can be performed securely.

The connector 43 is provided at the tip portion of the tube 41. The connector 43 has the male connector 6 along with the male lock section 8A. With this structure, the connector 43 can be connected to the connector 5A.

Since the connector 5A, the connector 43 and the infusion dosing section side connector 26 are each provided with at least one of the male lock section 8A and the female lock sections 9A, 9B, the connectors can be connected together without any possibility of becoming locked in a twisted state, which tends to be experienced by screw type connectors such as the conventional lure lock type. To be more specific, use of the aforementioned connection method ensures that, even when multiple infusion tubes are linked together, the infusion tubes can be aligned in a fixed direction and can be maintained easily.

In addition, a click (a sound confirming the connection) can be heard, for example, when the connection between the male lock section 8A of the connector 43 and the female lock section 9B of the connector 5A is completed (i.e., when the locked state is attained). This enables completion of a secure locking to be confirmed aurally as well, as contrasted with cases of screw type connectors, in which it is difficult to know how firmly the connectors should be fastened in order to achieve a locked state. In the following description of the connectors, and the connection formed therebetween, the connection between the male lock section 8A and the female lock section 9B shall be described representatively.

Further, in cases where fitting between the connectors is based on frictional contact between inclined parts (tapered parts), as in the case of lure connectors, the distance between the connectors upon fitting thereof is not constant. In such a case, therefore, the male lock section 8A and the female lock section 9B, which are disposed in parallel, must have extremely rigorous dimensional accuracy. On the other hand, since the connector 5A includes the valve element 51 (as described later), the elasticity of the valve element 51 provides an ample tolerance with respect to variations in distance between the connectors. Therefore, an assured locking (connection) providing a liquid-tight condition can be realized, without the need for rigorous dimensional accuracy.

Further, the connected condition of the male lock section 8A of the connector 43 and the female lock section 9B of the connector 5A, for example, can assume both a half-locked state and a locked state, as described above.

For example, until the second infusion tube 4a is placed in use, the male connector 6 of the connector 43 is loosely fitted at a position such that the valve element 51, disposed in the female connector 7B of the connector 5A, is not opened. In other words, until the second infusion tube 4a is used, the connector 43 remains connected to the connector 5A in a half-locked state. This ensures that the valve element 51 remains in a non-deformed state until just immediately before the valve element 51 allows the infusion to flow. Therefore, the valve element can effectively be put to use without deterioration in function.

In addition, in the locked state, the male connector 6 of the connector 43 and the female connector 7B of the connector 5A are connected in a liquid-tight manner, such that a liquid can securely flow from the second infusion tube 4a into the connector 5A.

Next, operation of (a method of using) the infusion tube set 1 shall be described below.

Herein, a description shall be made, by way of example, of the case in which the second infusion tube 4a is used as a first infusion line (first infusion route), for dosing a patient with an infusion, i.e., wherein the second infusion tube 4a is used mainly as an infusion tube through which a basic liquid or the like is fed, whereas the first infusion tube 4b is used as a second infusion line (second infusion route) for dosing the patient with an infusion, i.e., wherein the first infusion tube 4b is used mainly as an auxiliary route, or as an infusion tube, through which a lipid emulsion, a drug, an antibiotic agent or the like is fed to the patient.

When connecting the second infusion tube 4a, first, a maintaining infusion agent, for example, is prepared and placed in the infusion bag 31.

Next, the male connector 6 of the connector 43 is pushed into the female connector 7B of the connector 5A, in order to fit them together in a liquid-tight state.

Subsequently, as shown in FIG. 27, the bottle needle 451 of the second infusion tube 4a penetrates (pierces) the stopper (rubber stopper) of the infusion bag 31 that stores the infusion. As a result, the infusion bag 31 and the second infusion tube 4a are connected to each other through the bottle needle 451, creating a condition in which the infusion can be supplied from the infusion bag 31 to the second infusion tube 4a.

Next, priming of the passage inside the second infusion tube 4a is conducted.

Subsequently, the male connector 6 of the connector 5A of the second infusion tube 4a is inserted and fitted into the female connector 7A of the infusion dosing section side connector 26, which is connected to the outlet port 211 of the indwelling needle 21 that dwells within the blood vessel (e.g., a peripheral vein) 110 of the patient. As a result, the female connector 7A of the infusion dosing section side connector 26 and the male connector 6 of the connector 5A of the infusion tube 4a are connected in a liquid-tight manner. In this instance, the female lock section 9A of the infusion dosing section side connector 26 and the male lock section 8A of the connector 5A are placed in a locked state, so that the connection is prevented from easily being released.

Next, the slide forceps 46 for the second infusion tube 4a is operated in order to control the flow rate (dosing rate) of the infusion in the second infusion tube 4a to achieve an instructed flow rate (instructed dosing rate) for the maintaining infusion agent, wherein the patient is dosed with the infusion at the instructed dosing rate.

Incidentally, the infusion dosing section side connector 26 may be omitted, such that the male connector 6 of the connector 5A of the second infusion tube 4a is connected to the outlet port 211 of the indwelling needle 21.

Subsequently, when connecting the first infusion tube 4b, which is used for dosing the patient with, for example, an antibiotic agent at fixed time intervals, depending on the condition of the patient, a physiological saline solution containing the antibiotic agent dissolved therein is first prepared in an infusion bag 32.

Next, the male connector 6 of the connector 43 of the first infusion tube 4b is pushed into the female connector 7B of the connector 5A, to fit them together in a liquid-tight manner.

Subsequently, the bottle needle 451 of the first infusion tube 4b penetrates (pierces) a stopper (rubber stopper) of the infusion bag 32 that stores the infusion therein. As a result, the infusion bag 32 and the first infusion tube 4b are connected to each other via the bottle needle 451, resulting in a condition in which the infusion is supplied from the infusion bag 32 to the first infusion tube 4b side.

Next, priming of the passage inside the first infusion tube 4b is conducted.

Subsequently, as shown in FIGS. 28 and 29, the male connector 6 of the connector 5A of the first infusion tube 4b is inserted and fitted into the female connector 7A of the connector 5A of the second infusion tube 4a. As a result, the female connector 7A of the connector 5A of the second infusion tube 4a and the male connector 6 of the connector 5A of the first infusion tube 4b are connected in a liquid-tight manner. In this instance, the female lock section 9A of the second infusion tube 4a and the male lock section 8A of the first infusion tube 4b are placed in a locked state, such that the connection is prevented from easily being released (see FIG. 29).

Next, the slide forceps 46 for the first infusion tube 4b is operated in order to control the flow rate (dosing rate) of the infusion in the first infusion tube 4b to achieve an instructed flow rate (instructed dosing rate) for the antibiotic agent, and the patient is dosed with the infusion at the instructed dosing rate.

In this manner, the patient can be dosed (in mixture) with the maintaining infusion agent, via the second infusion tube 4a, and with the antibiotic agent-containing physiological saline, via the first infusion tube 4b.

In addition, in the event that another infusion line (infusion route) is installed, the male connector 6 of the connector 5A of another infusion tube (not shown) is inserted and fitted into the female connector 7A of the connector 5A of the first infusion tube 4b, in the same manner as described above. As a result, the female connector 7A of the connector 5A of the first infusion tube 4b and the male connector 6 of the connector 5A of the other infusion tube are connected in a liquid-tight manner.

Thereafter, any number of infusion lines can be added to the system, in the same manner as described above.

Incidentally, the methods for using the infusion tube set 1 described above are merely examples, and the invention is not limited to the above features.

For example, in the event that a patient is dosed with an infusion via the second infusion tube 4a, the same infusion may be increased in quantity by use of the first infusion tube 4b.

As has been described above, according to the infusion tube set 1, the ports (female connectors 7A, 7B and male connector 6) for connection of infusion tubes are always present, so that the number of infusion lines (infusion routes) can be increased speedily and assuredly.

In other words, since several ports for connection of infusion tubes are always present, it is possible to obviate a situation in which the number of ports for connection of infusion tubes becomes less than needed, for example, upon an abrupt change in the patient's condition.

In addition, each of the infusion lines is connectable in a liquid-tight manner.

Further, when adding a new infusion line, it suffices to insert the male lock section 8A of the connector 5A on one side into the female lock section 9A or 9B of the connector 5A on the other side. Therefore, installation of additional infusion lines can be conducted while dosing the patient with infusion(s) via already connected infusion tube(s) (for example, while dosing the patient with a minute quantity of drug(s)). This makes it possible to avoid risks, for example, when the concentration of drugs in the patient's blood is changed, causing changes in the patient's condition.

In addition, when adding a new infusion line, it is unnecessary to open the existing infusion line(s), or to reassemble and reconnect the infusion lines. This makes it possible to avoid risks such as increasing the possibility of misconnecting the infusion line(s) or introducing infections due to bacteria.

Incidentally, in the present invention, the number of infusion tube(s) employed in the infusion tube set may also be one, or may be three or more.

Further, in the present invention, in the event that the infusion tube set has a plurality of infusion tubes, the infusion tubes may all be the same, or the infusion tubes may all be different from each other, or any portion of them may be the same.

### <Second Embodiment>

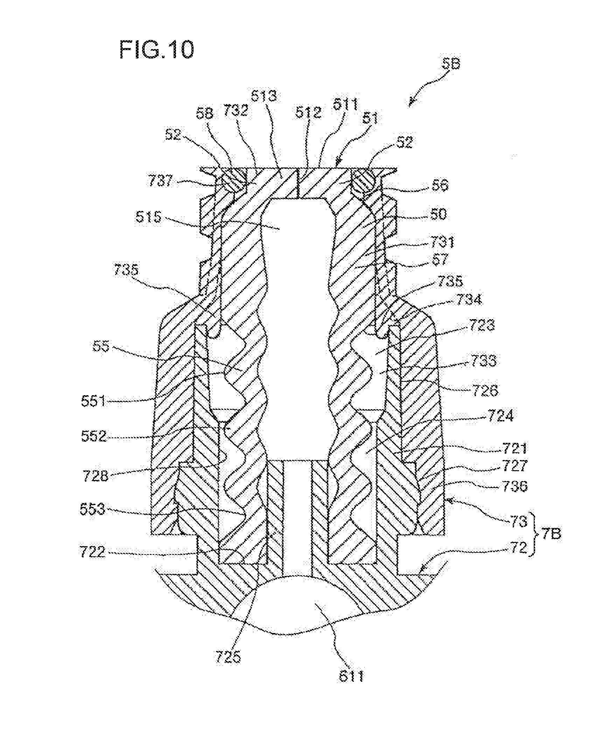

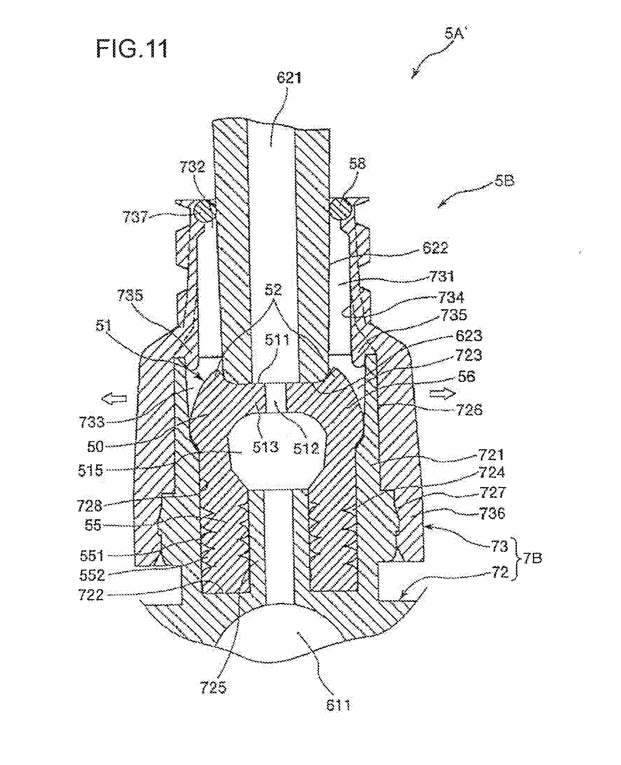

FIGS. 10 and 11 are longitudinal sectional views showing a second embodiment of the connector of the present invention.

The second embodiment of the connector according to the present invention shall be described below with reference to the figures. The following description shall be centered on differences between the present embodiment and the aforementioned embodiment, wherein descriptions of the same items, which are common to both embodiments, shall be omitted.

This embodiment is the same as the first embodiment described above, except for the provision of the seal member.

As shown in FIGS. 10 and 11, at a connection port 732 (first cavity 731) of a female connector 7B of a connector 5B, an O-ring (seal member) 58 is fixed along an inner peripheral surface 734 of the connection port 732. The O-ring 58 has a circular vertical cross-sectional shape.

The inner peripheral surface 734 of the connection port 732 of the female connector 7B is provided with a ring-shaped recess 737 arranged along the circumferential direction thereof. The O-ring 58 is fitted (fixed) into the recess 737.

In addition, the vertical cross-sectional shape of the recess 737 enables easy and secure fitting of the O-ring 58 into the recess 737, namely, the recess 737 is substantially semi-circular in shape.

With the O-ring 58 thus provided, in the locked state, a lure tapered part 622 of a male connector 6 of a connector 5A' can make close contact with the inside of the O-ring 58 (see FIG. 11). In addition, in the locked state, an opening part 623 of the male connector 6 of the connector 5A' is maintained in close contact with a top face 511 of a valve element 51, which is similar to the case of the first embodiment described above.

This configuration ensures that the male connector 6 of the connector 5A' and the female connector 7B of the connector 5B can be connected securely in a liquid-tight manner.

Incidentally, the cross-sectional shape of the O-ring 58 is not limited to being a circle. For example, the shape may be an ellipse, a tetragon, or the like.

In addition, the material used for forming the O-ring 58 is not particularly limited. For example, the materials already mentioned above in connection with the description of the valve element 51 can be used.

In addition, although the connector 5B also includes the valve element 51, the invention is not limited to such a configuration, and the valve element 51 may be omitted if desired. Even if the valve element 51 is omitted, the lure tapered part 622 of the male connector 6 of the connector 5A' can still come into close contact with the O-ring 58, as mentioned above, and therefore, the male connector 6 of the connector 5A' and the female connector 7B of the connector 5B can be connected together securely in a liquid-tight manner.

### <Third Embodiment>

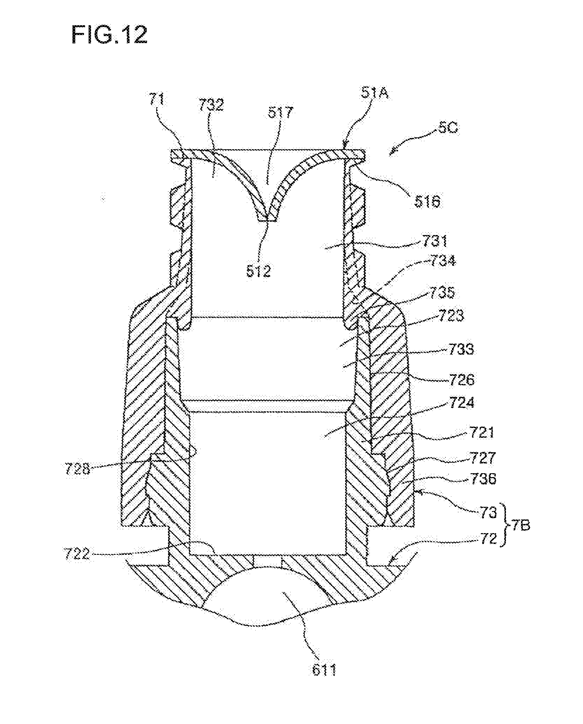

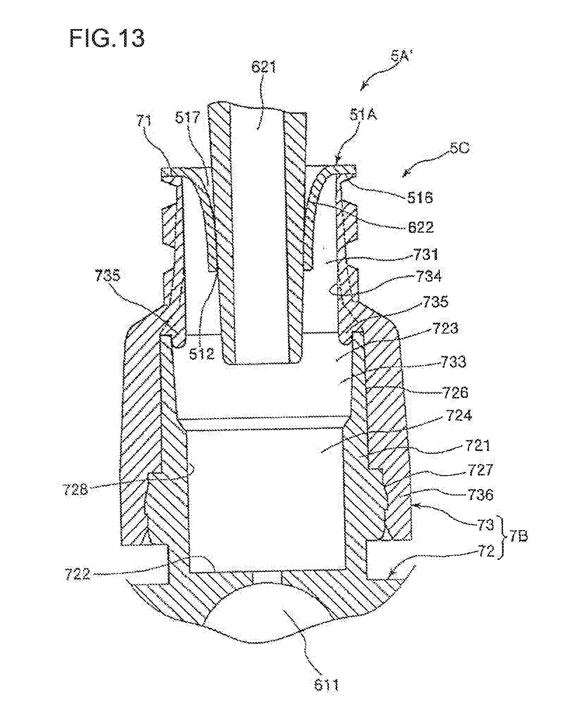

FIGS. 12 and 13 are longitudinal sectional views showing a third embodiment of the connector according to the present invention.

The third embodiment of the connector according to the present invention shall be described below with reference to the figures. The following description shall be centered on differences between the present embodiment and the aforementioned embodiments, wherein descriptions of the same items, which are common to the embodiments, shall be omitted.

This embodiment is the same as the first embodiment described above, except for the provision of the seal member.

As shown in FIGS. 12 and 13, in the connector 5C, a valve element 51, similar to that disposed in the connector 5A of the first embodiment, has been omitted. In addition, a valve element (seal member) 51A is fixed to an opening part 71 of a female connector 7B of the connector 5C.

The valve element 51A includes a fixed section 516 having an edge part fixed to the opening part 71, and a convergent section 517 having a shape that converges while projecting toward a first cavity 731 of the female connector 7B. Further, the convergent section 517 is provided with a slit 512 formed in a crest part thereof. The valve element 51A, thus configured, is in the form of a so-called duckbill valve.

With the valve element 51A, in a locked state, a lure tapered part 622 of a male connector 6 of a connector 5A' can open the slit 512 in the valve element 51A, i.e., the lure tapered part 622 can push open the convergent section 517. In addition, due to the elastic force (restoring force) of the pushed-open convergent section 517 itself, the convergent section 517 can come into close contact with the lure tapered part 622 (see FIG. 13).

This ensures that the male connector 6 of the connector 5A' and the female connector 7B of the connector 5B can be securely connected together in a liquid-tight manner.

Incidentally, the material used for forming the valve element 51A is not particularly limited. For example, the materials already mentioned above in connection with the description of the valve element 51 can be used.

In addition, while the valve element 51 has been omitted in the connector 5C, the present invention is not limited to such a configuration. For example, the valve element 51 may be provided, in the same manner as in the connector 5A according to the first embodiment.

### <Fourth Embodiment>

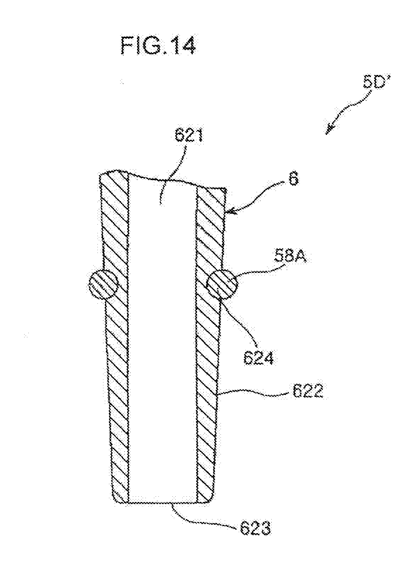

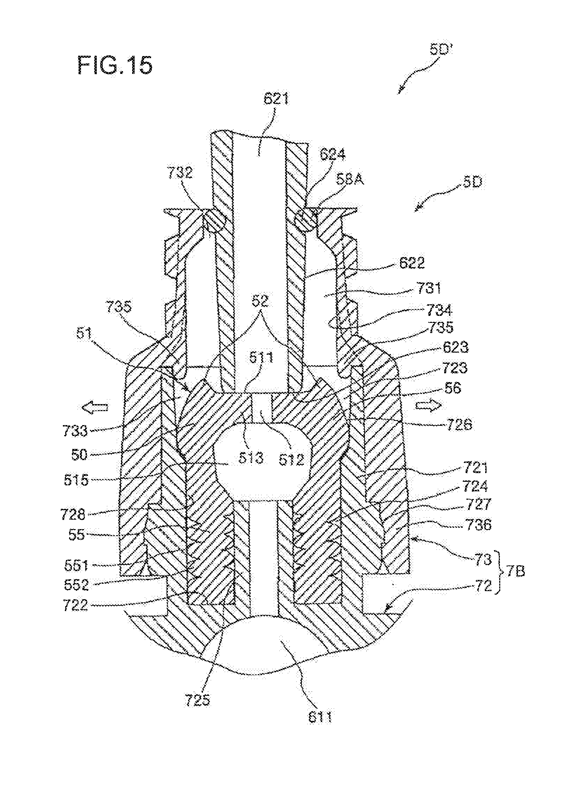

FIGS. 14 and 15 are longitudinal sectional views showing a fourth embodiment of the connector according to the present invention.

Next, the fourth embodiment of the connector according to the present invention shall be described below with reference to the figures. The following description shall be centered on differences between the present embodiment and the aforementioned embodiments, wherein descriptions of the same items, which are common to the embodiments, shall be omitted.

This embodiment is the same as the first embodiment above, except for the provision of the seal member.

The connector 5D in this embodiment has an O-ring (seal member) 58A fixed to the outer peripheral side of a male connector 6. The O-ring 58A has a circular vertical cross-sectional shape.

In addition, a male connector 6 of a connector 5D' can be connected to a female connector 7B of the connector 5D.

Herein, the term "connector 5D'" refers to a connector having the male connector 6, in a manner similar to that of the connector 5D. Namely, the connector 5D' has, fixed to the male connector 6 thereof, an O-ring 58A, which is similar to that of the male connector 6 of the connector 5D (see, for example, FIG. 14).

The male connector 6 (lure tapered part 622) shown in FIG. 14 is provided, at an intermediate portion thereof, with a ring-shaped recess 624 arranged in the circumferential direction. The O-ring 58A is fitted (fixed) into the recess 624.

In addition, the vertical cross-sectional shape of the recess 624 enables the O-ring 58A to be fitted easily and securely in the recess 624, i.e., the recess 624 has a substantially semi-circular shape.

With the O-ring 58A, in a locked state, the O-ring 58A of the male connector 6 of the connector 5D' can come into close contact with an edge part (inner peripheral surface 734) of a connection port 732 of the female connector 7B of the connector 5D (see FIG. 15). In addition, in the locked state, an opening part 623 of the male connector 6 of the connector 5D' is placed in close contact with a top face 511 of a valve element 51.

This configuration ensures that the male connector 6 of the connector 5D' and the female connector 7B of the connector 5D can be connected together more securely in a liquid-tight manner.

Incidentally, the vertical cross-sectional shape of the O-ring 58A is not limited to being a circle. For example, the shape may be an ellipse, a tetragon, or the like.

Further, the material used for forming the O-ring 58A is not particularly limited. For example, the materials already mentioned above in connection with the description of the valve element 51 can be used.

In addition, although the connector 5D includes the valve element 51, the invention is not limited to such a configuration, and the valve element 51 may be omitted if desired. Even if the valve element 51 is omitted, the O-ring 58A of the male connector 6 of the connector 5D' can still make close contact with an edge portion of the connection port 732 of the female connector 7B of the connector 5D, and therefore, the male connector 6 of the connector 5D' and the female connector 7B of the connector 5D can be connected securely in a liquid-tight manner.

### <Fifth Embodiment>

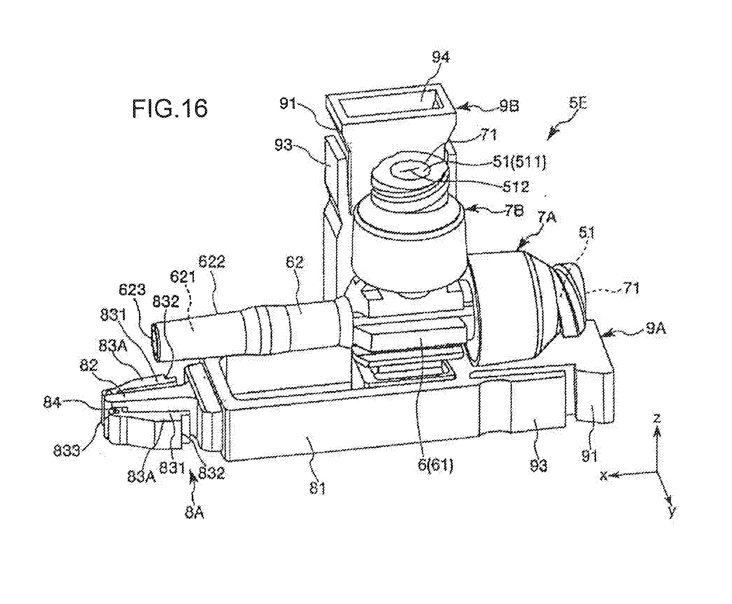

FIG. 16 is a perspective view showing a fifth embodiment of the connector according to the present invention.

The fifth embodiment of the connector according to the present invention shall be described below with reference to FIG. 16. The following description shall be centered on differences between the present embodiment and the aforementioned embodiments, wherein descriptions of the same items, which are common to the embodiments, shall be omitted.

The fifth embodiment is the same as the first embodiment described above, except for the outer shape of the connector.

As shown in FIG. 16, a connector 5E includes a male lock section 8A disposed in the z-axis negative direction in relation to a male connector 6. In addition, the connector 5E includes a female lock section 9A disposed in the z-axis negative direction in relation to a female connector 7A.

The connector 5E configured (shaped) as described above can, for example, be easily placed in a locked state with another connector 5E, which is similar to the connector 5E, in the same manner as the connector 5A of the first embodiment. In addition, the female connectors 7A, 7B of the connector 5A and the male connector 6 of the other connector 5E can be connected securely in a liquid-tight manner.

Incidentally, the connector intended for connection to the connector 5E is not limited to the other connector 5E. For example, the connector 5A or the connector 5A' may also be used.

### <Sixth Embodiment>

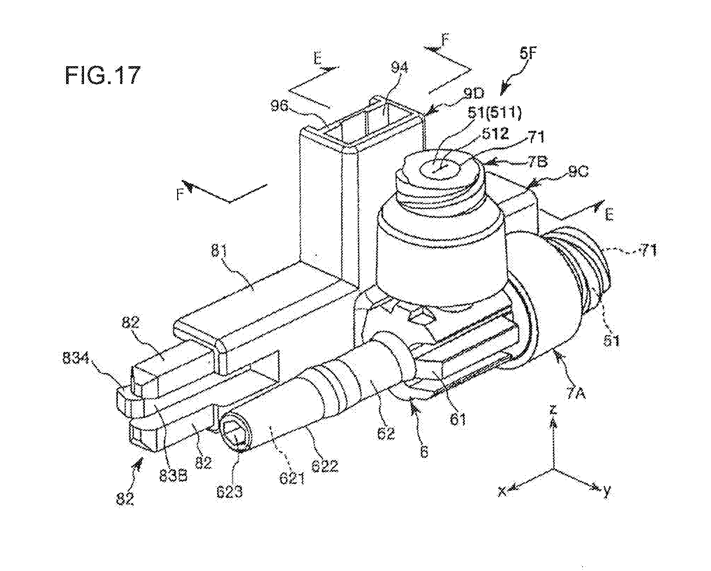

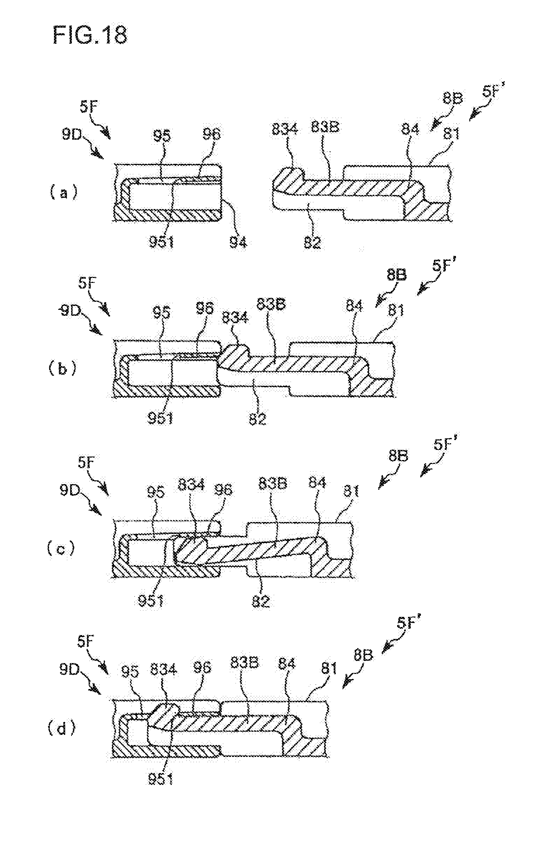

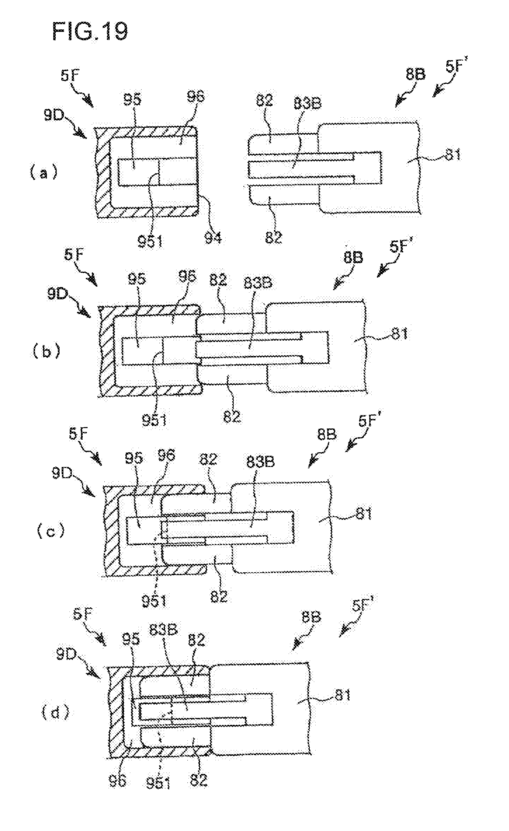

FIG. 17 is a perspective view showing a sixth embodiment of the connector according to the present invention. FIG. 18 illustrates sectional views taken along line E-E of FIG. 17, whereas FIG. 19 illustrates sectional views taken along line F-F of FIG. 17.

Next, the sixth embodiment of the connector according to the present invention shall be described below with reference to the figures. The following description shall be centered on differences between the present embodiment and the aforementioned embodiments, wherein descriptions of the same items, which are common to the embodiments, shall be omitted.

The present embodiment is the same as the first embodiment above, except for the configurations of the male lock and female lock sections.

As shown in FIG. 17, a connector 5F includes a male connector 6, female connectors 7A and 7B, a male lock section 8B, female lock sections 9C and 9D, and a valve element 51. Incidentally, FIGS. 18 and 19 respectively illustrate the connection steps used for connecting the connector 5F and a connector 5F', from a state prior to their connection until they are connected together a locked state.

Herein, the term "connector 5F'" refers to a connector having a male connector 6 and a male lock section 8B, similar to those of the connector 5F.

The male lock section 8B includes a base (male lock body) 81, a pair of projecting parts 82, 82 projecting from the base 81, a claw part 83B disposed between the projecting parts 82, and an energizing part 84 for energizing the claw part 83B in the y-axis negative direction.

As shown in FIGS. 17 and 19, the claw part 83B projects in the x-axis positive direction from one end of the base 81. The claw part 83B is provided at one end thereof with a male-side engaging part 834, which projects in the y-axis negative direction (see FIG. 18).

The female lock sections 9C and 9D make up portions to which the male lock section 8B of the connector 5F' can be coupled.

The female lock section 9C is disposed in the x-axis negative direction in relation to the base 81 (male lock section 8B). In addition, the female lock section 9D is disposed in the z-axis positive direction in relation to the base 81 (male lock section 8B). Since the female lock sections 9C and 9D are substantially the same in shape (configuration), only the female lock section 9D shall be described below as representative of both.

As shown in FIG. 18, the female lock section 9D is provided with a female-side engaging part 95, which engages with a male-side engaging part 834 of the male lock section 8B of the connector 5F'. The female-side engaging part 95 penetrates into a portion of a wall part 96, in the y-axis negative direction of the female lock section 9D.

As shown in FIG. 18(d), an edge portion (end) 951 of the female-side engaging part 95 of the female lock section 9D engages with the male-side engaging part 834 of the male lock section 8B of the connector 5F', whereby the connector 5F (female lock section 9D) and the connector 5F' (male lock section 8B) are placed in a locked state.

The connection process (locking state) of the connector 5A and the connector 5A' shall be described below.

Starting from a condition in which the connector 5F and the connector 5F' are spaced from each other (see FIG. 18(a) and FIG. 19(a)), the male lock section 8B of the connector 5F' is brought closer to the female lock section 9D of the connector 5F (see FIG. 18(b) and FIG. 19(b)).

As the male lock section 8B of the connector 5F' is inserted into the female lock section 9D of the connector 5F, as shown in FIG. 18(c), a wall portion 96 of the female lock section 9D of the connector 5F presses against the male-side engaging part 834 (claw part 83B) of the connector 5F', against an energizing force of the energizing part 84 of the connector 5F'.

When the male lock section 8B of the connector 5F' is pushed further into the female lock section 9D of the connector 5F, as shown in FIG. 18(d), the male-side engaging part 834 of the connector 5F' engages with the female-side engaging part 95 of the connector 5F, i.e., the connector 5F' and the connector 5F are placed in a locked state.

In addition, in the locked state, when the connector 5F' is pressed by the male connector 6, the valve element 51 of the connector 5F becomes deformed, thereby opening the slit 512 in the valve element 51. As a result, the male connector 6 of the connector 5F' and the female connector 7B of the connector 5F are connected, while enabling liquid to flow therethrough.

Further, the locked state of the connector 5F' and the connector 5F can be released by pressing the male-side engaging part 834 of the male lock section 8B of the connector 5F' against the energizing force of the energizing part 84. In other words, starting from the locked state shown in FIG. 18(d), the male-side engaging part 834 of the male lock section 8B of the connector 5F' is pressed. As a result, engagement between the female-side engaging part 95 of the connector 5F and the male-side engaging part 834 of the connector 5F' is cleared (released).

Thereafter, when the connector 5F' is pulled outward, the connector 5F' and the connector 5F are spaced away from each other.

Thus, the connector 5F and the connector 5F' can be connected to each other perfectly, i.e., the connectors 5F and 5F' can be securely placed in a locked state, by a simple operation of pushing in the male lock section 8B of the connector 5F' into the female lock section 9D of the connector 5F.

In addition, substantially at the same time that the locked state is attained, the female connector 7B of the connector 5F and the male connector 6 of the connector 5F' are securely connected in a liquid-tight state.

Further, since the male lock section 8B of the connector 5F' is inserted into the female lock section 9D of the connector 5F, as shown in FIGS. 19(c) and 19(d), outer peripheral surfaces of the projecting parts 82 slide along the inner peripheral surface of the female lock section 9D. This facilitates guiding of the male lock section 8B of the connector 5F' into the female lock section 9D of the connector 5F. Therefore, the center axis of the male lock section 8B and the center axis of the female lock section 9D can be made to coincide with each other, whereby the projecting parts 82 function as guides.

### <Seventh Embodiment>

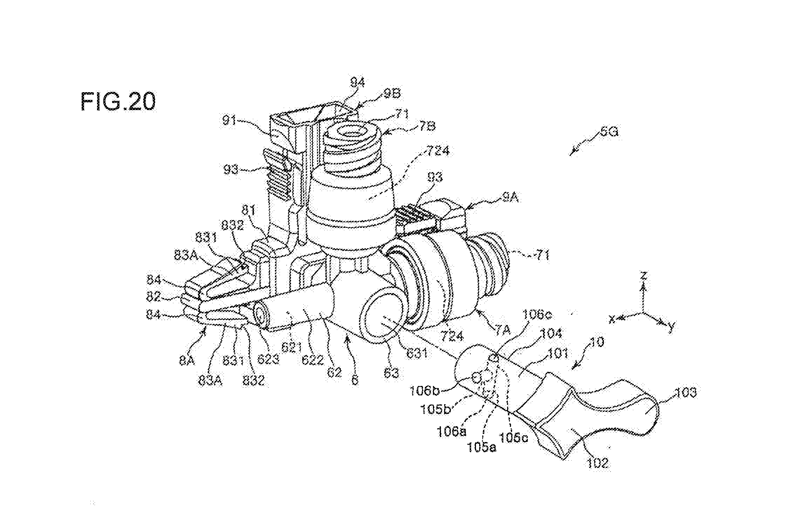

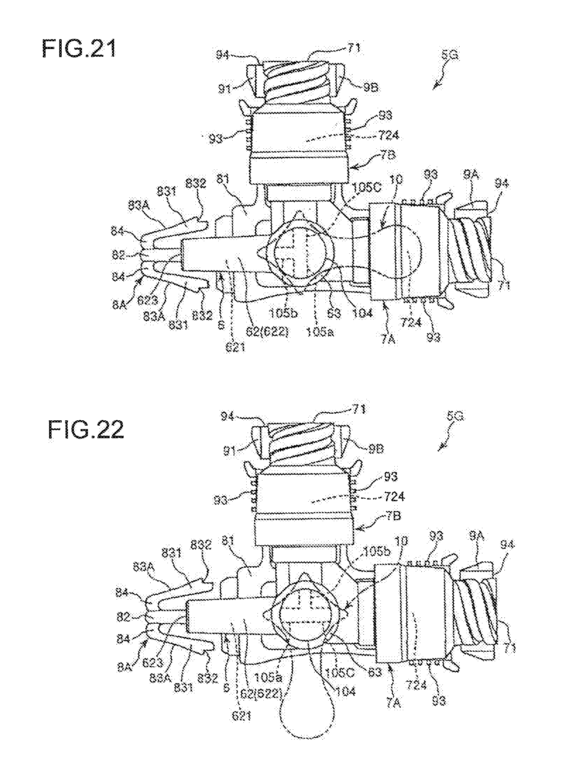

FIG. 20 is a perspective view showing a seventh embodiment of the connector according to the present invention. FIGS. 21 to 24 are side views showing a passage switching pattern in the connector shown in FIG. 20.

The seventh embodiment of the connector according to the present invention shall be described below with reference to the figures. The following descriptions shall be centered on differences between the present embodiment and the aforementioned embodiments, wherein descriptions of the same items, which are common to the embodiments, shall be omitted.

This embodiment is the same as the first embodiment described above, except that the connector further includes a cock, which serves as a passage switching means.

The connector 5G shown in FIG. 20 includes a hollow cylindrical part 63 formed at an intersection portion where the center axis of a male connector 6 (or the center axis of a female connector 7A) and the center axis of a female connector 7B intersect.

The hollow cylindrical part 63 is formed so as to project in the y-axis positive direction. In addition, a cavity 631 in the hollow cylindrical part 63 communicates with a liquid passage 621 in a male connector 6, a third cavity 724 in the female connector 7A, and a third cavity 724 in the female connector 7B.

The connector 5G thus constructed further includes a cock 10, serving as a passage switching means, for switching the passage(s) for the liquid(s) that flow in the connector 5G.

As shown in FIG. 20, the cock 10 includes a barrel part 101, a base part 102, and a lever 103.

The barrel part 101 has a solid cylindrical or a hollow cylindrical shape (a solid cylindrical shape is shown in FIG. 1), and is rotatably inserted (fitted) into the cavity 631 of the hollow cylindrical part 63, in a gas-tight or liquid-tight manner. Therefore, the outer diameter of the barrel part 101, upon pulling the cock 10 out of the hollow cylindrical part 63, is preferably larger than the inside diameter of the hollow cylindrical part 63, for example, by about 1% to 6%.

In addition, the outer peripheral surface 104 of the barrel part 101 is smooth.

The barrel part 101 is provided with passages 105a, 105b, and 105c therein, corresponding respectively to the liquid passage 621 in the male connector 6, the third cavity 724 in the female connector 7A, and the third cavity 724 in the female connector 7B. The passages 105a, 105b, and 105c are formed in a T-shaped pattern. Specifically, the three passages 105a, 105b, and 105c extend in radial directions of the barrel part 101 at angular intervals of 90°, and are formed so as to communicate with each other at an intermediate position within the barrel part 101.

Further, the passages 105a to 105c are formed at positions coinciding respectively with the liquid passage 621 in the male connector 6, the third cavity 724 in the female connector 7A, and the third cavity 724 in the female connector 7B, in a condition wherein the cock 10 is fitted into the hollow cylindrical part 63 (hereinafter referred to simply as a "cock fitted condition").

The passages 105a to 105c open toward the outer peripheral surface 104 of the barrel part 101, to form circular openings 106a, 106b, and 106c, respectively.

Incidentally, the diameters of the openings 106a to 106c are preferably equal to the inside diameters of the passages 105a to 105c. Therefore, since the diameters of the openings 106a to 106c are large, a greater flow rate can be assured.

Further, the respective diameters of the openings 106a to 106c are preferably substantially equal to each other, although they may be different if desired.

In addition, peripheral edge portions (profiles) of the openings 106a to 106c may be chamfered beforehand, in which case a chipping preventative effect on the peripheral edge portions or the vicinity thereof is further enhanced.

At an end in the y-axis positive direction of the barrel part 101, the base part (lever support part) 8, which has a cross-section greater than that of the barrel part 101, is formed. Preferably, the base part (lever support part) 8 is formed as one body with the barrel part 101. In the cock fitted condition, the base part 102 is exposed at an end of the hollow cylindrical part 63.

At the outer periphery of the base part 102, a lever 103 is formed, which projects while extending in a direction opposite to the passage 105b. Preferably, the lever 103 is formed as one body with the base part 102. The cock 10 is turned by gripping the lever 103 with the fingers, and applying a torque thereto.

Incidentally, the lever 103 for performing the turning operation is not limited to a lever that extends in one direction as shown in the figure. The lever may also extend in two or more directions, or may be a lever that is similar to a handle.

While the cock 10 can be freely turned 360° in relation to the hollow cylindrical part 63 (male connector and female connector) in this embodiment, the invention is not limited to this configuration. Specifically, the connector 5G may be provided with a restricting means (not shown), which restricts the angular range by which the cock 10 can be turned relative to the hollow cylindrical part 63. Such a restricting means can be composed, for example, of projected portions, which are formed respectively at an end portion of the hollow cylindrical part 63, as well as at the base part 102, and which can engage with each other in the cock fitted condition.

The material forming the cock 10 is not particularly limited. For example, materials similar to the materials used for forming the connector 5A, exclusive of the valve element 51, can be used.

Next, operations of the connector 5G shall be described below with reference to FIGS. 21 through 24.

When the position of the lever 103 of the cock 10 is set in the same direction as the female connector 7A, as shown in FIG. 21, the liquid passage (cavity) 621 in the male connector 6 and the third cavity 724 in the female connector 7B communicate with each other through the passages 105b and 105c formed in the barrel part 101 of the cock 10, whereas the third cavity 724 in the female connector 7A is sealed by the outer peripheral surface 104 of the barrel part 101. As a result, the male connector 6 and the female connector 7B are in a state of communication, whereas the female connector 7A is in a closed state.

When the position of the lever 103 of the cock 10 is set in a direction 180° opposite to the female connector 7B, as shown in FIG. 22, the liquid passage 621 in the male connector 6, the third cavity 724 in the female connector 7A, and the third cavity 724 in the female connector 7B communicate with each other through the passages 105a, 105b and 105c formed in the barrel part 101 of the cock 10. As a result, the male connector 6 and the female connectors 7A and 7B are in a state of communication.

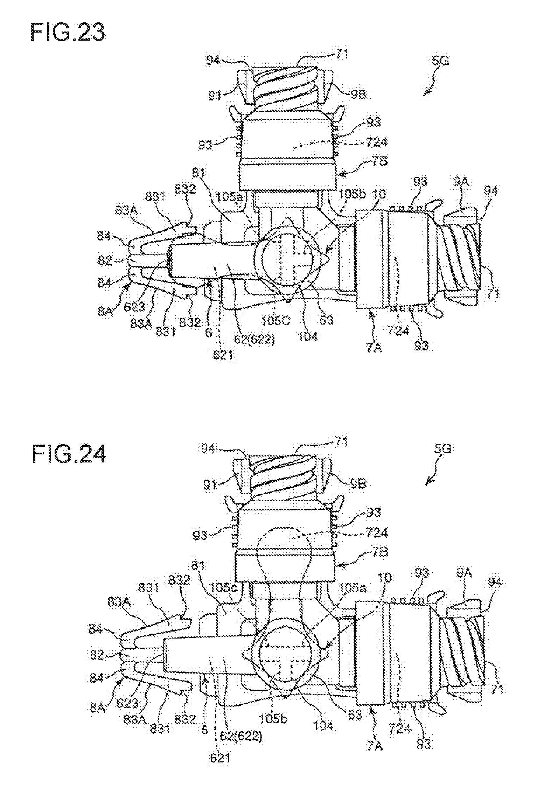

When the position of the lever 103 of the cock 10 is set in the same direction as the male connector 6, as shown in FIG. 23, the third cavity 724 in the female connector 7A and the third cavity 724 in the female connector 7B communicate with each other through the passages 105a and 105b formed in the barrel part 101 of the cock 10, whereas the liquid passage 621 in the male connector 6 is sealed by the outer peripheral surface 104 of the barrel part 101. As a result, the female connector 7A and the female connector 7B are in a state of communication, whereas the male connector 6 is in a closed state.

When the position of the lever 103 of the cock 10 is in the same direction as the female connector 7B, as shown in FIG. 24, the liquid passage 621 in the male connector 6 and the cavity 724 in the female connector 7A communicate with each other through the passages 105a and 105c formed in the barrel part 101 of the cock 10, whereas the cavity 724 in the female connector 7B is sealed by the outer peripheral surface 104 of the barrel part 101. As a result, the male connector 6 and the female connector 7A are in a state of communication, whereas the female connector 7B is in a closed state.

Thus, in the connector 5G, by turning the cock 10, opening and closing of the male connector 6 (liquid passage 621), the female connector 7A (third cavity 724) and the female connector 7B (third cavity 724), can be appropriately selected.

Effects of the above-described connector 5G shall now be described, referring, by way of example, to a case in which an infusion bag storing an infusion therein, which is desired to constantly (continuously) flow toward the male connector 6, is connected to the female connector 7A. Another infusion bag storing an infusion therein, which is desired to intermittently flow toward the male connector 6, is connected to the female connector 7B.

By setting the cock 10 in the state shown in FIG. 24, the infusion can be made to constantly flow from the female connector 7A into the male connector 6. In this instance, the female connector 7B is sealed, making it impossible for the infusion to flow from the female connector 7B into the male connector 6.

As a result of turning the cock 10, from the state shown in FIG. 24 to the state shown in FIG. 22, the infusion can be made to flow from the female connector 7A into the male connector 6, in the same manner as shown in FIG. 22, and further, the infusion can be made to flow from the female connector 7B into the male connector 6.

Thus, passages for the liquids flowing in the connector 5G can be switched by turning the cock 10. Accordingly, for example, it is possible to mix two kinds of liquids together at an arbitrary timing.

Incidentally, although the valve element 51 has been omitted in the structure of the connector 5G shown in the figures, the valve element 51 may be provided if desired.

### <Eighth Embodiment>

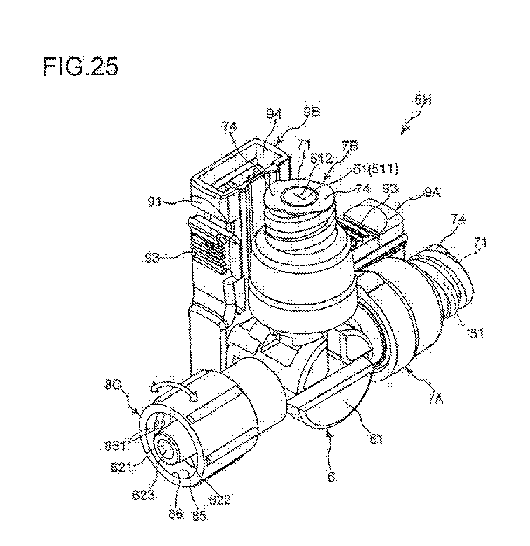

FIG. 25 is a perspective view showing an eighth embodiment of the connector according to the present invention.

The eighth embodiment of the connector according to the present invention shall be described below with reference to FIG. 25. The following description shall be centered on differences between the present embodiment and the aforementioned embodiments, wherein descriptions of the same items, which are common to the embodiments, shall be omitted.

The present embodiment is the same as the first embodiment above, except for the structure of the male lock section.

In the connector 5H shown in FIG. 25, a male lock section 8C forms a lure lock. Specifically, the male connector 8C includes a tubular ring member, which is disposed on an outer peripheral side of a male connector 6 concentrically with the male connector 6, so as to be (coaxially) rotatable therewith. The tubular ring member further is provided with a screw thread 851, formed on an inner peripheral part 85 thereof.

In addition, at an edge portion (outer peripheral portion) of an opening part 71 of each of the female connectors 7A and 7B of the connector 5H, a pair of projecting parts 74, 74 are formed, which project in opposite radial directions from the opening part 71.

In the locked state, the projecting parts 74 can engage with a screw thread 851 of a male lock section 8C, possessed by another connector similar to the connector 5H. Thus, each of the projecting parts 74 forms a female lock section, which can be coupled to a male lock section 8C.

For example, when the female connector 7B of the connector 5H is connected to the male connector 6 of the other connector, first, the projecting parts 74 of the female connector 7B of the connector 5H engage with an opening part of the male lock section 8C of the other connector.

Next, in the engaged state described immediately above, the male lock section 8C of the other connector is turned in a direction so as to bring the connector 5H and the other connector closer to each other. Owing to such a turning operation, the male connector 6 of the other connector enters into the female connector 7B of the connector 5H, via the opening part 71 of the female connector 7B.

Then, the male lock section 8C of the other connector is turned further, whereby a portion of a lure tapered part 622 of the male connector 6 of the other connector is placed in liquid-tight contact with an inner peripheral portion of the opening part 71 of the female connector 7B of the connector 5H (see, for example, FIG. 9).

Thus, by means of the connector 5H and the other connector, the above-mentioned operations enable the projecting parts 74 of the connector 5H and the male lock section 8C of the other connector to be brought securely into a locked state, whereby the female connector 7B of the connector 5H and the male connector 6 of the other connector are securely connected in a liquid-tight manner.

### <Ninth Embodiment>

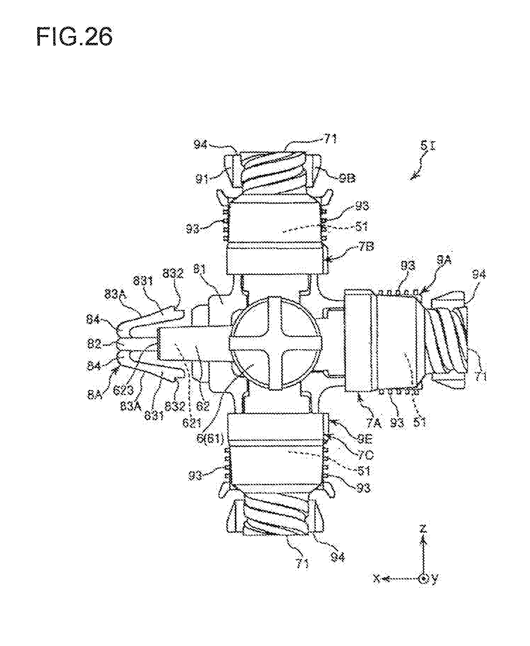

FIG. 26 is a side view showing a ninth embodiment of the connector according to the present invention.

The ninth embodiment of the connector according to the present invention shall be described below with reference to FIG. 26. The following description shall be centered on differences between the present embodiment and the aforementioned embodiments, wherein descriptions of the same items, which are common to the embodiments, shall be omitted.

This embodiment is the same as the first embodiment above, except for the number of female connectors that are disposed.

The connector 5I shown in FIG. 26 also includes a female connector 7C, which is configured in substantially the same manner as the female connectors 7A and 7B, together with a female lock section 9E, which is configured in substantially the same manner as the female lock sections 9A and 9B.

The female connector 7C is disposed in the z-axis negative direction. Therefore, the female connectors 7A, 7B and 7C are laid out such that adjacent female connectors are orthogonal to each other.

In addition, the female lock section 9E is disposed in the z-axis negative direction, similar to the female connector 7C. The female lock section 9E is also adjacent to, and disposed in the y-axis negative direction, relative to the female connector 7C.

In the connector 5I, opening parts 71 of the female connectors 7A, 7B and 7C open respectively in three different directions, specifically, in the x-axis negative direction, in the z-axis positive direction, and in the z-axis negative direction.

This ensures, for example, that when the number of infusion lines is increased, as in the first embodiment, the three tubes constituting the liquid passages extend in three different directions, thereby preventing the tubes from interfering with each other. In addition, the flow directions of the liquids flowing through the connector 5I can be switched.

### <Tenth Embodiment>

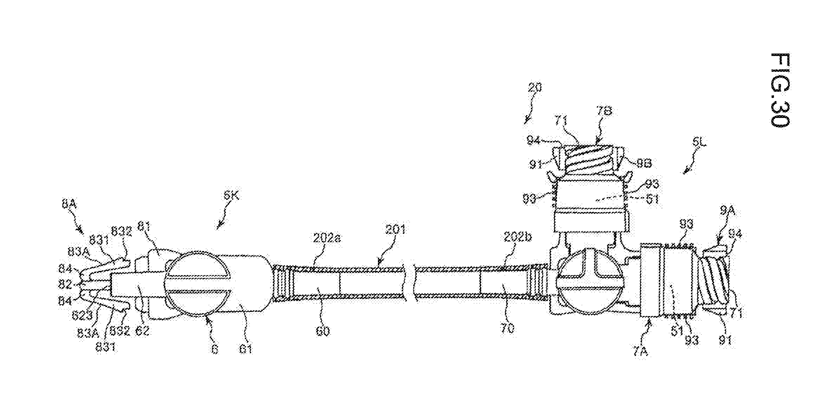

FIG. 30 is a partial longitudinal sectional view of a tube assembly according to the present invention.

An embodiment of the tube assembly according to the present invention (tenth embodiment) shall be described below with reference to FIG. 30. The following description shall be centered on differences between the present embodiment and the aforementioned embodiments, wherein descriptions of the same items, which are common to the embodiments, shall be omitted.

The tube assembly 20 shown in FIG. 30 includes a flexible tube 201, and connectors 5K and 5L, which are connected respectively to ends 202a and 202b of the tube 201.

The tube 201 permits a liquid flowing inwardly via the connector 5K on one side to flow toward the connector 5L on the other side.

Incidentally, the material used for forming the tube 201 is not particularly limited. Examples of appropriate materials include polyolefins such as polyethylene, polypropylene, ethylene-vinyl acetate copolymer, etc., polyvinyl chloride, polybutadiene, polyamides, polyesters, etc., wherein among such materials, polybutadiene is particularly preferred. When polybutadiene is used as the material for forming the tube 201, the tube 201 has appropriate flexibility and chemical resistance, along with excellent chemical adsorption preventative properties.

The connector 5K has a male connector 6 and a male lock section 8A, which are substantially the same as those of the connector 5A according to the first embodiment, and a connection section 60 that communicates with the male connector 6. The connection section 60 is tubular in shape, and projects in the direction opposite to the projecting direction of the male connector 6.

In addition, an end 202a of the tube 201 is connected to the connection section 60. This ensures that the male connector 6 communicates with the tube 201 through the connection section 60.

The connector 5L includes female connectors 7A and 7B, which are substantially the same as those of the connector 5A according to the first embodiment, female lock sections 9A and 9B, which are substantially the same as those of the connector 5A of the first embodiment, and a connection section 70 communicating with third cavities 724 in the female connectors 9A and 9B. The connection section 70 is tubular in shape, and projects in a direction opposite to the projecting direction of the female connector 9A.

Further, an end 202b of the tube 201 is connected to the connection section 70. This ensures that the third cavities 724 in the female connectors 9A and 9B communicate with the tube 201 through the connection section 70.

Connection of the tube assembly 20, for example, to an infusion tube set, as described in the first embodiment above, results in a tube assembly 20 that constitutes a portion of the liquid passages, such that the liquid passages of the infusion tube set can easily be extended.

In addition, the connectors 5K and 5L enable the tube assembly 20 and the infusion tube set to be placed in a locked state, whereby the tube assembly 20 is securely prevented from becoming released from the infusion tube set unintentionally. Further, the connectors 5K and 5L ensure that the tube assembly 20 and the infusion tube set are connected together securely in a liquid-tight manner, such that the liquid(s) therein can be securely prevented from leaking undesirably.

Incidentally, the tube assembly 20 is not limited to one in which the connectors 5K and 5L are connected respectively to the ends 202a and 202b of the tube 201. For example, a connector may be connected to only one of the ends 202a and 202b. Further, for example, in the case that the connector 5K is connected to the end 202a, a female lure connector may be connected to the end 202b. Alternatively, in the case that a hollow centesis needle is connected to the end 202a, the connector 5L may be connected to the end 202b. The centesis needle may be used for centesis of the surface of an organism, or for puncturing (piercing) a rubber stopper, which closes the mouth of a bag (container) in which a liquid is stored.

In addition, although the connector 5L includes two female connectors 9A and 9B in the configuration shown in the figure, the invention is not limited to such a configuration. For example, the connector 5L may consist of only one such female connector.

### <Eleventh Embodiment>

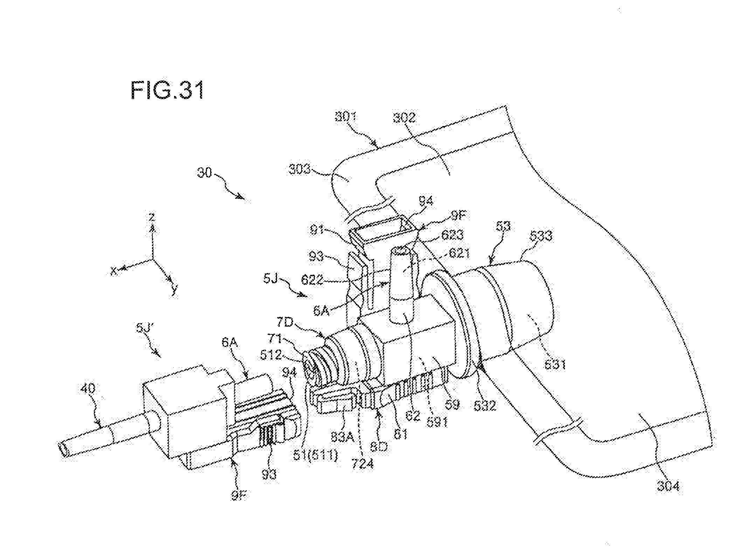

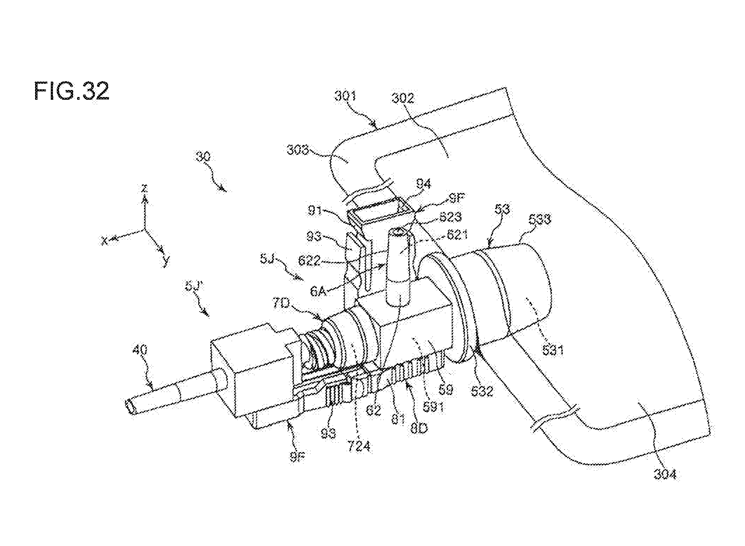

FIGS. 31 and 32 are perspective views of a container for medical use according to the present invention.

Next, an embodiment (eleventh embodiment) of the container for medical use according to the present invention shall be described below with reference to the figures. The following description shall be centered on differences between the present embodiment and the aforementioned embodiments, wherein descriptions of the same items, which are common to the embodiments, shall be omitted.

The container 30 for medical use, as shown in FIGS. 31 and 32, includes a container body 301 suitable for containing a liquid therein, and a connector 5J, which is fixed to the container body 301. Incidentally, the container body 301 shall be described below, taking an infusion bag as an example thereof.

The connector 5J includes a connector body 59, a male connector 6A disposed in the z-axis positive direction in relation to the connector body 59, a female connector 7D disposed in the x-axis positive direction in relation to the connector body 59, a male lock section 8D disposed in the z-axis negative direction in relation to the connector body 59, a female lock section 9F disposed in the y-axis negative direction in relation to the connector body 59, and a connection section 53 disposed in the x-axis negative direction in relation to the connector body 59.

The connector body 59 has a block-like outer shape, and is formed with a cavity 591 therein.

The male connector 6A is substantially the same in structure as the male connector 6 of the connector 5A according to the first embodiment above. The male connector 6A projects in the z-axis positive direction. In addition, a liquid passage 621 formed in the male connector 6A communicates with the cavity 591 in the connector body 59.

The female connector 7D is substantially the same in structure as the female connectors 7A and 7B of the connector 5A according to the first embodiment above. The female connector 7D projects in the x-axis positive direction. Further, a third cavity 724 formed in the female connector 7D communicates with the cavity 591 in the connector body 59.

The male lock section 8D is substantially the same in structure as the male lock section 8A of the connector 5A according to the first embodiment above. The male lock section 8D is disposed adjacent to the female connector 7D, and projects in the x-axis positive direction, in the same manner as the female connector 7D.

The female lock section 9F is substantially the same in structure as the female lock sections 9A and 9B of the connector 5A according to the first embodiment above. The female lock section 9F is disposed adjacent to the male connector 6A, and projects in the z-axis positive direction, in the same manner as the male connector 6A.

The connection section 53 is tubular in shape, and includes a cavity (hollow portion) 531 therein that communicates with the cavity 591 in the connector body 59. The connection section 53 is fixed to the container body 301 of the connector 5J.

In addition, an outer peripheral part of the connection section 53 is provided with a flange 531 thereon, which is formed by enlarging a portion of the outer peripheral part in diameter. When the connector 5J is fixed to the container body 301, the flange 531 contacts the container body 301 from the outside, whereby the connector 5J is securely prevented from entering excessively into the container body 301, i.e., the connector 5J can be positioned properly in relation to the container body 301.

Further, the connection section 53 is provided, on an outer peripheral portion thereof, with a tapered part 533, wherein the outside diameter of the outer peripheral portion decreases gradually in the x-axis negative direction. For instance, when no liquid is contained inside the container body 301, a gap is formed between the tapered part 533 and the inside surface of the container body 301, so that inside surfaces of the container body 301 can be prevented from adhering to each other. This permits liquid to be easily injected into or discharged from the container body 301.

The container body 301 is made up of two flexible resin-formed sheet members 302, 302.

In order to form the container body 301, edge parts 303 of the sheet members 302 are joined together, for example, by fusing (heat fusing, microwave fusing, ultrasonic fusing, or the like). A space 304 is formed between the two sheet members 302, 302, wherein a liquid can be contained within the space 304.

Incidentally, the material forming the sheet members 302 is not particularly limited. Examples of suitable materials include various resin materials, such as polyethylene, polypropylene, polycarbonate, etc.

The connector 5J is fixed to a portion of the edge parts 303. As a result, the connector 5J forms a port through which liquid is discharged from the container body 301. Incidentally, the method by which the connector 5J is fixed is not particularly limited. Examples may include fusing (heat fusing, microwave fusing, ultrasonic fusing, etc.) and adhesion (adhesion using an adhesive or a solvent).

As shown in FIG. 31 (and also in FIG. 32), the connector 5J is connected, which is fixed to the container body 301.

Herein, the term "connector 5J'" implies a connector having a male connector 6A and a female lock section 9F, which are similar to those of the connector 5J. In addition, a tube 40 that communicates with the liquid passage 621 in the male connector 6A of the connector 5J' is connected to a side of the connector 5J' opposite to the male connector 6A.

Further, the male connector 6A and the female lock section 9F of the connector 5J' are disposed such that they correspond, respectively, to the female connector 7D and the male lock section 8D of the connector 5J.

As shown in FIG. 32, when the connector 5J of the container 30 is employed for medical use and the connector 5J' is connected therewith, the male lock section 8D of the connector 5J and the female lock section 9F of the connector 5J' are connected securely in a locked state, and moreover, the female connector 7D of the connector 5J and the male connector 6A of the connector 5J' are connected securely in a liquid-tight manner. This permits liquid in the container body 301 to pass sequentially through the connection section 53, the connector body 59, and the female connector 7D of the connector 5J, as well as through the male connector 6A of the connector 5J' and through the tube 40.

In addition, an operating part 93, which functions to release the locked state of both the male lock section 8D of the connector 5J and the female lock section 9F of the connector 5J', is disposed on the side of the connector 5J'. Therefore, the connector 5J' is gripped when the locked state is released. This effectively prevents the connector 5J' from flying out in the x-axis positive direction, under a repulsive force (restoring force) of a valve element 51 in the connector 5J, when the locked state is released.

Incidentally, the liquid contained in the container body 301 is not particularly limited. Examples of such liquids include blood derivatives, carbohydrate injections such as glucose, electrolyte correcting injections such as sodium chloride and potassium lactate, vitamin preparations, vaccines, antibiotic injections, imaging contrast mediums, steroid preparations, proteolytic enzyme inhibitors, lipid emulsions, various protein preparations, and various liquid medicines such as carcinostatics, anesthetics, stimulants, narcotics, etc., various diagnostic medicines, as well as other liquids such as distilled water, physiological saline, disinfectants, nutrition preparations, liquid foods, alcohols, etc.

In addition, while an infusion bag has been mentioned as one example of the container body 301 according to this embodiment, the invention is not limited to this feature. For example, a blood transfusion bag, a cultivation container, a waste liquid container, or the like, may also be used as the container body 301.

Further, in place of or in addition to the port composed of the connector 5J, the container body 301 may be provided with, for example, a port that is plugged with a rubber stopper.

In addition, although the connector 5J is a port that is intended for use when a liquid is discharged from the liquid container 301, the invention is not limited to this feature. The connector 5J may also be a port that is used when a liquid is injected into the liquid container 301.

Further, the tapered part 533 of the connection section 53 of the connector 5J may be provided with a side hole (slit) communicating with the cavity 531. When the tapered part 533 is provided with such a side hole, for example, liquid inside the container body 301 can be discharged assuredly via the side hole.

In addition, when the tapered part 533 is provided with a side hole therein, the side hole is preferably formed so as to open in the y-axis direction.

Although the connector, the tube assembly, the infusion tube set, and the container for medical use according to the present invention have been described above on the basis of particular embodiments illustrated in the drawings, the present invention is not limited to these embodiments. The components and configurations thereof can be replaced by other arbitrary configurations having the same or equivalent functions.

Incidentally, in the present invention, any combination of two or more configurations (characteristics) of the above-described embodiments may be employed in combination. Further, other arbitrary components can be added to the present invention.

In addition, in the connector employed in the infusion tube set according to the present invention, any combination of two or more configurations (characteristics) of the above-described embodiments may be combined.

For example, in the connector according to the sixth embodiment, the male lock section may be oriented in the z-axis negative direction in relation to the male connector, in the same manner as in the connector according to the fifth embodiment.

Further, for example, in the connector according to the fifth embodiment, an O-ring similar to that employed in the connector of the second embodiment, or an O-ring similar to that employed in the connector of the fourth embodiment, may be provided.

In addition, for example, in the connector according to the fifth embodiment, the valve element disposed in the female connector may be omitted, and in its place, a valve element (duckbill valve) similar to that employed in the connector of the third embodiment may be provided.

Further, for example, in the connector according to the sixth embodiment, an O-ring similar to that employed in the connector of the second embodiment, or an O-ring similar to that employed in the connector of the fourth embodiment, may be provided.

In addition, for example, in the connector according to the sixth embodiment, the valve element disposed in the female connector may be omitted, and in its place, a valve element (duckbill valve) similar to that employed in the connector of the third embodiment may be provided.

Further, in each of the connectors according to the second to sixth embodiments, as well as those according to the eighth to eleventh embodiments, a passage switching means similar to the passage switching means (cock) of the seventh embodiment may be provided.

In addition, in each of the connectors according to the second to seventh embodiments, as well as those according to the ninth to eleventh embodiments, the male lock section in each of the connectors may be similar in configuration to the male lock section employed in the seventh embodiment.

In addition, in each of the connectors according to the second to eighth embodiments, as well as those according to the tenth and eleventh embodiments, three female connectors with opening parts thereof that open in different directions may be provided, in the same manner as in the connector of the ninth embodiment.

In addition, any of the connectors of the second to ninth embodiments may be used as connectors in the tube assembly according to the tenth embodiment. In addition, the two connectors in the tube assembly of the tenth embodiment may be the same or different from each other.

In addition, although in the connectors of the first to tenth embodiments the male connector and the male lock section correspond to each other, whereas the female connector and the female lock section correspond to each other, the invention is not limited to this feature. For example, as in the case of the connector in the container for medical use according to the eleventh embodiment, the male connector and the female lock section may correspond to each other, whereas the female connector and the male lock section may correspond to each other.

### INDUSTRIAL APPLICABILITY

The connector according to the present invention includes a male connector having a cavity and a female connector having a cavity, to which another male connector similar to the male connector can be connected, wherein the connector includes: a male lock section; and a female lock section, to which another male lock section similar to the male lock section can be coupled. When the connector is in a locked state, in which the female lock section and the other male lock section are coupled, the female connector and the other male connector are connected so that the cavities thereof communicate with each other, enabling a liquid to flow therethrough. Therefore, the locked state can be attained easily and assuredly, and further, the liquid can flow assuredly in the locked state. Accordingly, the centesis implement according to the present invention exhibits industrial applicability.

## Claims

1. A connector comprising a male connector having a cavity and a female connector having a cavity, to which another male connector similar to said male connector can be connected,
wherein said connector includes:
a male lock section; and
a female lock section, to which another male lock section similar to said male lock section can be coupled; and
when said connector is in a locked state, in which said female lock section and said other male lock section are coupled, said female connector and said other male connector are connected so that the cavities thereof communicate with each other enabling a liquid to flow therethrough.

2. The connector according to claim 1, wherein when said female connector and said other male connector are located close to each other, said female lock section and said other male lock section are set in a half-locked state.

3. The connector according to claim 1, wherein when the locked state is released, flow of said liquid is intercepted.

4. The connector according to claim 1,
wherein said male lock section has a claw part provided with a male-side engaging part, and an energizing part for energizing said claw part;
said female lock section has a female-side engaging part, which is engageable with a male-side engaging part of said other male lock section similar to said male lock section; and
said connector is set in said locked state through engagement of said female-side engaging part with said male-side engaging part of said other male lock section, and said locked state is released when said male-side engaging part of said other male lock section is pushed against an energizing force of said energizing part.

5. The connector according to claim 1,
wherein said male lock section has a pair of claw parts, which are provided with a first male-side engaging part and which can be moved toward and away from each other, and an energizing part for energizing said claw parts away from each other;
said female lock section has a first female-side engaging part, which is engageable with a first male-side engaging part of said other male lock section similar to said male lock section, and an operating part which is operable to bring claw parts of said other male lock section closer to each other when said first female-side engaging part and said first male-side engaging part of said other male lock section engage with each other; and
said connector is set in said locked state through engagement of said first female-side engaging part with said first male-side engaging part of said other male lock section.

6. The connector according to claim 5,
wherein said claw parts comprise a second male-side engaging part;
said female lock section comprises a second female-side engaging part; and
a half-locked state, in which said female connector and said other male connector are located close to each other, is obtained by engagement of said second female-side engaging part with a second male-side engaging part of said other male lock section.

7. The connector according to claim 1,
wherein said male lock section comprises a tubular ring member, which is disposed at an outer peripheral side of said male connector, so as to be rotatable concentrically with said male connector, and comprising a screw thread on an inner peripheral part;
said female connector comprises, on an outer peripheral part thereof, a projection that engages with a screw thread of said other male lock section similar to said male lock section; and
said connector is set in said locked state through engagement of said projection with said screw thread of said other male lock section.

8. The connector according to claim 1,
wherein said male connector and said male lock section are disposed adjacent to each other, such that projecting directions thereof are parallel to each other; and
said female connector and said female lock section are disposed adjacent to each other, such that projecting directions thereof are parallel to each other.

9. The connector according to claim 1,
wherein said connector comprises a plurality of said female connectors; and
at least one of said female connectors and said male connector are disposed such that center lines thereof are substantially orthogonal to each other.

10. The connector according to claim 1,
wherein said connector comprises a plurality of said female connectors; and
at least one of said female connectors and said male connector are disposed such that center lines thereof are parallel to each other, and an opening part of said female connector and an opening part of said male connector are oriented oppositely to each other.

11. The connector according to claim 1, wherein said connector comprises a plurality of said female connectors, and opening parts of said female connectors open in different directions.

12. The connector according to claim 1, further comprising a seal member formed from an elastic material, which keeps a liquid-tight connection between said female connector and said other male connector when said connector is in said locked state.

13. The connector according to claim 12, wherein said seal member is disposed in said cavity of said female connector, said seal member comprising a surface in close contact with an end portion of said other male connector when said connector is in said locked state, and a slit formed in said surface and which is open when said connector is in said locked state.

14. The connector according to claim 12, wherein said seal member is disposed in an opening part of said female connector, said seal member having a convergent section that converges while projecting toward said cavity of said female connector.

15. The connector according to claim 12, wherein said seal member is annular in shape, said seal member being disposed on an inner peripheral surface of said cavity of said female connector and along a circumferential direction of said cavity.

16. The connector according to claim 12, wherein said seal member is annular in shape, said seal member being disposed on an outer peripheral side of said male connector and along a circumferential direction of said male connector.

17. The connector according to claim 1, wherein said male connector(s) and said female connector(s) total three or more in number, and further comprising passage switching means for selecting arbitrary two cavities from among said cavities of said male and female connectors and switching liquid passages therein such that said selected two cavities communicate with each other.

18. The connector according to claim 17, wherein said passage switching means comprises a cock, which is turnable relative to said male and female connectors, and which is formed with passages therein corresponding respectively to said cavities, wherein opening and closing of said cavities is selected by turning said cock.

19. A connector comprising a male connector having a cavity, and to which a female connector having a cavity can be connected,
wherein said connector has a male lock section or a female lock section, to which another male lock section similar to said male lock section can be coupled; and
when said connector is in a locked state in which said female lock section and said other male lock section are coupled to each other, or when said connector is in a locked state in which said male lock section and another female lock section similar to said male lock section are coupled, said male connector and said female connector are connected such that said cavities thereof communicate with each other so as to enable a liquid to flow therethrough.

20. A connector comprising a female connector having a cavity, and to which a male connector having a cavity can be connected,
wherein said connector has a male lock section or a female lock section, to which another male lock section similar to said male lock section can be coupled; and
when said connector is in a locked state in which said female lock section and said other male lock section are coupled to each other, or when said connector is in a locked state in which said male lock section and another female lock section similar to said male lock section are coupled, said female connector and said male connector are connected such that said cavities thereof communicate with each other so as to enable a liquid to flow therethrough.

21. A tube assembly comprising:
a flexible tube; and
a connector as set forth in any of claims 1 to 20, connected to at least one end of said tube.

22. An infusion tube set comprising:
at least one connector as set forth in any of claims 1 to 20;
a tube assembly which forms a passage for an infusion, one end of which is connectable to said connector;
a connection section provided at the other end of said tube assembly, and which is connected to a side of a containing section containing said infusion; and
an infusion dosing section comprising an infusion dosing section side connector connectable to said male connector or to said female connector of said connector, and which is operative to dose a patient with said infusion.

23. A container for medical use, comprising a flexible container body in which a liquid can be contained, and a connector as set forth in any of claims 1 to 20 fixed to said container body,
wherein said connector forms a port through which said liquid is injected into and/or discharged from said container body.
